# EUROPEAN PATENT APPLICATION

(11) **EP 4 570 801 A1**
(43) Date of publication of application: **18.06.2025**
(21) Application number: 23852284.1
(22) Date of filing: 06.07.2023
(51) Int. Cl.: C07D 471/04, C09K 11/06, G09F 9/30, H01L 29/786, H10K 50/10, H10K 50/16, H10K 10/40, H10K 30/50, H10K 59/00, H10K 85/10

(54) **PHENANTHROLINE COMPOUND, AND ORGANIC THIN FILM AND ORGANIC SEMICONDUCTOR ELEMENT USING SAME**

(30) Priority: 09.08.2022 JP 2022127078
(71) Applicant: Nippon Shokubai Co., Ltd., Osaka-shi, Osaka 541-0043 (JP)
(72) Inventor: HASEGAWA, Munehiro, Suita-shi, Osaka 564-0034 (JP); FUKUDOME, Hiroki, Suita-shi, Osaka 564-0034 (JP); OSHIMA, Kazuyuki, Suita-shi, Osaka 564-0034 (JP); SHIOZAKI, Yoko, Suita-shi, Osaka 564-0034 (JP)
(74) Representative: Cabinet Beau de Loménie
(86) International application number: PCT/JP2023/025169
(87) International publication number: WO 2024/034301

(57) **Abstract**

The present invention provides a phenanthroline compound and an organic thin film including the phenanthroline compound each capable of providing excellent electron injection properties and electron transport properties when used in an electron injection layer of an organic electroluminescent device. The present invention relates to a phenanthroline compound represented by the following formula (1): 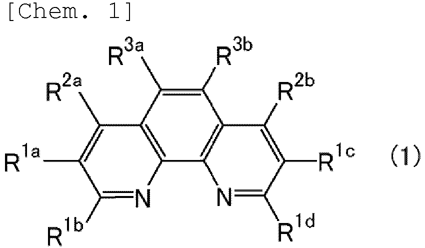 wherein R^{1a} to R^{1d} each independently represent a hydrogen atom, an aromatic hydrocarbon group optionally having a substituent, or an aromatic heterocyclic group optionally having a substituent; R^{2a} and R^{2b} each independently represent a dialkylamino group optionally having a substituent, and the dialkylamino group optionally having a substituent may form a ring structure; R^{3a} and R^{3b} each independently represent a hydrogen atom, an aromatic hydrocarbon group optionally having a substituent, an aromatic heterocyclic group optionally having a substituent, -R⁴=R⁵-R⁶ (where R⁴ and R⁵ each represent CH or N and R⁶ represents a hydrogen atom, an aromatic hydrocarbon group optionally having a substituent, or an aromatic heterocyclic group optionally having a substituent), or - R⁷-R⁸ (where R⁷ represents CH₂, NH, O, or S and R⁸ represents a hydrogen atom, an aromatic hydrocarbon group optionally having a substituent, or an aromatic heterocyclic group optionally having a substituent), or R^{3a} and R^{3b} are bonded to each other to form a fused ring structure; and at least one of R^{1a} to R^{1d}, R^{3a}, and R^{3b} is an aromatic hydrocarbon group optionally having a substituent or an aromatic heterocyclic group optionally having a substituent.

## Description

### TECHNICAL FIELD

The present invention relates to phenanthroline compounds and organic thin films using the same. Specifically, the present invention relates to an organic semiconductor device such as an organic electroluminescent device or an organic thin-film solar cell, containing a phenanthroline compound.

### BACKGROUND ART

Organic electroluminescent devices can provide thin, soft, and flexible displays. Moreover, displays including organic electroluminescent devices can display higher-brightness, higher-definition images than currently dominant liquid crystal displays and plasma displays. Moreover, displays including organic electroluminescent devices have wider viewing angles than liquid crystal displays. Such displays including organic electroluminescent devices are expected to be more widely used in the future as, for example, displays for televisions and mobile phones.

Organic electroluminescent devices are also expected to be used as lighting systems.

An organic electroluminescent device includes a stack including a cathode, a light-emitting layer, and an anode. In an organic electroluminescent device, the energy difference between the work function of the anode and the highest occupied molecular orbital (HOMO) of the light-emitting layer is smaller than the energy difference between the work function of the cathode and the lowest unoccupied molecular orbital (LUMO) of the light-emitting layer. Thus, electron injection from the cathode into the light-emitting layer is more difficult than hole injection from the anode into the light-emitting layer. In response to this, in existing organic electroluminescent devices, an electron injection layer is disposed between the cathode and the light-emitting layer to facilitate electron injection from the cathode to the light-emitting layer.

Patent Literature 1 discloses an organic electroluminescent device including an organic thin film made of a phenanthroline derivative at the interface between an electrode and an organic layer.

### CITATION LIST

### - Patent Literature

Patent Literature 1: WO 2021/045179

### SUMMARY OF INVENTION

### - Technical Problem

Organic electroluminescent devices including an existing electron injection layer however do not have sufficient electron injection properties and electron transport properties and require further improved electron injection properties and electron transport properties.

The present invention has been made in view of the above circumstances and aims to provide a phenanthroline compound and an organic thin film including the phenanthroline compound each capable of providing excellent electron injection properties and electron transport properties when used in an electron injection layer of an organic electroluminescent device.

The present invention also aims to provide a phenanthroline compound having excellent electron injection properties and electron transport properties; an organic semiconductor device and an organic electroluminescent device each including an organic thin film containing the phenanthroline compound of the present invention; a display and a lighting system each including the organic electroluminescent device; an organic thin-film solar cell; and an organic thin-film transistor.

### - Solution to Problem

The present inventors have conducted various investigations to achieve the above object and have arrived at the present invention.

Specifically, a first aspect of the present invention relates to a phenanthroline compound represented by the following formula (1): 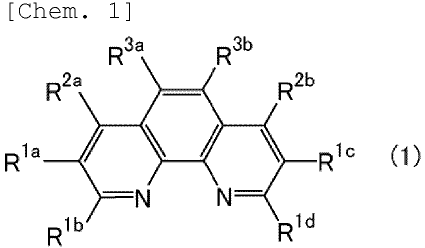 wherein R^{1a} to R^{1d} each independently represent a hydrogen atom, an aromatic hydrocarbon group optionally having a substituent, or an aromatic heterocyclic group optionally having a substituent; R^{2a} and R^{2b} each independently represent a dialkylamino group optionally having a substituent, and the dialkylamino group optionally having a substituent may form a ring structure; R^{3a} and R^{3b} each independently represent a hydrogen atom, an aromatic hydrocarbon group optionally having a substituent, an aromatic heterocyclic group optionally having a substituent, -R⁴=R⁵-R⁶ (where R⁴ and R⁵ each represent CH or N and R⁶ represents a hydrogen atom, an aromatic hydrocarbon group optionally having a substituent, or an aromatic heterocyclic group optionally having a substituent), or - R⁷-R⁸ (where R⁷ represents CH₂, NH, O, or S and R⁸ represents a hydrogen atom, an aromatic hydrocarbon group optionally having a substituent, or an aromatic heterocyclic group optionally having a substituent), or R^{3a} and R^{3b} are bonded to each other to form a fused ring structure; and at least one of R^{1a} to R^{1d}, R^{3a}, and R^{3b} is an aromatic hydrocarbon group optionally having a substituent or an aromatic heterocyclic group optionally having a substituent.

A second aspect of the present invention relates to an organic thin film containing the phenanthroline compound represented by the formula (1).

A third aspect of the present invention relates to an organic semiconductor device material containing the phenanthroline compound represented by the formula (1).

A fourth aspect of the present invention relates to the organic semiconductor device material according to the third aspect of the present invention, wherein the organic semiconductor device material is for use in an organic semiconductor device selected from the group consisting of an organic electroluminescent device, an organic transistor, a photoelectric conversion device, and an organic solar cell.

A fifth aspect of the present invention relates to an organic semiconductor device containing the phenanthroline compound represented by the formula (1).

A sixth aspect of the present invention relates to the organic semiconductor device according to the fifth aspect of the present invention, wherein the organic semiconductor device is selected from the group consisting of an organic electroluminescent device, an organic transistor, a photoelectric conversion device, and an organic solar cell.

A seventh aspect of the present invention relates to an image display or lighting fixture including the organic semiconductor device according to the fifth or sixth aspect of the present invention.

### - Advantageous Effects of Invention

The phenanthroline compound of the present invention is a material having excellent electron injection properties and electron transport properties. The phenanthroline compound also has excellent heat resistance. Thus, organic electroluminescent devices or organic thin-film solar cells containing such a phenanthroline compound can have excellent durability, and the phenanthroline compound can be preferably used as an organic semiconductor device material.

### BRIEF DESCRIPTION OF DRAWINGS

FIG. 1 is a schematic cross-sectional view illustrating an example of an organic EL device of the present invention.
FIG. 2 is a schematic cross-sectional view illustrating an example of the organic EL device of the present invention.

### DESCRIPTION OF EMBODIMENTS

The present invention is described in detail below.

A combination of two or more of individual preferred embodiments of the present invention described below is also a preferred embodiment of the present invention.

### Phenanthroline compound of the present invention

The phenanthroline compound of the present invention is a phenanthroline compound represented by the following formula (1): 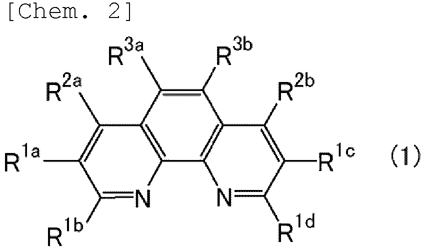 wherein R^{1a} to R^{1d} each independently represent a hydrogen atom, an aromatic hydrocarbon group optionally having a substituent, or an aromatic heterocyclic group optionally having a substituent; R^{2a} and R^{2b} each independently represent a dialkylamino group optionally having a substituent, and the dialkylamino group optionally having a substituent may form a ring structure; R^{3a} and R^{3b} each independently represent a hydrogen atom, an aromatic hydrocarbon group optionally having a substituent, an aromatic heterocyclic group optionally having a substituent, -R⁴=R⁵-R⁶ (where R⁴ and R⁵ each represent CH or N and R⁶ represents a hydrogen atom, an aromatic hydrocarbon group optionally having a substituent, or an aromatic heterocyclic group optionally having a substituent), or - R⁷-R⁸ (where R⁷ represents CH₂, NH, O, or S and R⁸ represents a hydrogen atom, an aromatic hydrocarbon group optionally having a substituent, or an aromatic heterocyclic group optionally having a substituent), or R^{3a} and R^{3b} are bonded to each other to form a fused ring structure; and at least one of R^{1a} to R^{1d}, R^{3a}, and R^{3b} is an aromatic hydrocarbon group optionally having a substituent or an aromatic heterocyclic group optionally having a substituent.

Examples of the aromatic hydrocarbon groups for R^{1a} to R^{1d}, R^{3a}, R^{3b}, R⁶, and R⁸ include C6-C30 aromatic hydrocarbon groups such as groups each formed by removing one hydrogen atom from an aromatic ring of a compound selected from a compound including only one aromatic ring (e.g., benzene); a compound in which two or more aromatic rings are directly bonded to each other via a single carbon atom of each aromatic ring (e.g., biphenyl or diphenylbenzene); and a fused-ring aromatic hydrocarbon compound (e.g., naphthalene, anthracene, phenanthrene, pyrene, or fluorene). Of these, the compound in which two or more aromatic rings are directly bonded to each other via a single carbon atom of each aromatic ring and the fused-ring aromatic hydrocarbon compound each preferably include 2 to 5 aromatic rings, more preferably 2 or 3 aromatic rings.

Examples of the aromatic heterocyclic groups for R^{1a} to R^{1d}, R^{3a}, R^{3b}, R⁶, and R⁸ include C3-C30 aromatic heterocyclic groups such as groups each formed by removing one hydrogen atom from an aromatic heterocycle of a compound selected from a compound including only one aromatic heterocycle (e.g., thiophene, furan, pyrrole, oxazole, oxadiazole, thiazole, thiadiazole, imidazole, pyridine, pyrimidine, pyrazine, or triazine); a compound in which two or more of these compounds including only one aromatic heterocycle are directly bonded to each other via a single carbon atom of each compound (e.g., bipyridine); a compound in which at least one of these compounds including only one aromatic heterocycle and at least one compound including only one aromatic ring (e.g., benzene) are directly bonded to each other via a single carbon atom of each compound (e.g., phenylpyridine); and a fused-ring heteroaromatic hydrocarbon compound (e.g., quinoline, quinoxaline, benzothiophene, benzothiazole, benzimidazole, benzoxazole, indole, carbazole, dibenzofuran, dibenzothiophene, acridine, or phenanthroline).

Of these, the compound in which two or more of these compounds including only one aromatic heterocycle are directly bonded to each other via a single carbon atom of each compound, the compound in which at least one of these compounds including only one aromatic heterocycle and at least one compound including only one aromatic ring (e.g., benzene) are directly bonded to each other via a single carbon atom of each compound, and the fused-ring heteroaromatic hydrocarbon compound each preferably include 2 to 5 aromatic rings, more preferably 2 or 3 aromatic rings.

The aromatic hydrocarbon groups and aromatic heterocyclic groups for R^{1a} to R^{1d}, R^{3a}, R^{3b}, R⁶, and R⁸ and the dialkylamino group for R² may each have one or two or more monovalent substituents. Examples of the monovalent substituents include a hydroxy group; a halogen atom such as a fluorine atom; a haloalkyl group such as a fluoromethyl group, a difluoromethyl group, or a trifluoromethyl group; a linear or branched C1-C20 alkyl group such as a methyl group, an ethyl group, a propyl group, an isopropyl group, a butyl group, an isobutyl group, or a tert-butyl group; a C5-C7 cyclic alkyl group such as a cyclopentyl group, a cyclohexyl group, or a cycloheptyl group; a linear or branched C1-C20 alkoxy group such as a methoxy group, an ethoxy group, a propoxy group, an isopropoxy group, a butoxy group, an isobutoxy group, a tert-butoxy group, a pentyloxy group, a hexyloxy group, a heptyloxy group, or an octyloxy group; a nitro group; a cyano group; an alkylamino group having a C1-C10 alkyl group, such as a methylamino group, an ethylamino group, a dimethylamino group, or a diethylamino group; a cyclic amino group such as a pyrrolidino group, a piperidino group, or a morpholino group; a diarylamino group such as a diphenylamino group or a carbazolyl group; an acyl group such as an acetyl group, a propionyl group, or a butyryl group; a C2-C30 alkenyl group such as a styryl group; a C5-C20 aryl group optionally substituted with a halogen atom (e.g., a fluorine atom), a C1-C20 alkyl or alkoxy group, or an amino group (specific examples of the aryl group are the same as those of the aromatic hydrocarbon groups described above); a C4-C20 heterocyclic group containing at least one selected from a nitrogen atom, a sulfur atom, and an oxygen atom and optionally substituted with a halogen atom (e.g., a fluorine atom), a C1-C20 alkyl or alkoxy group, or an amino group (the heterocyclic group may be one including only one ring, may be a compound in which two or more compounds including only one aromatic heterocycle are directly bonded to each other via a single carbon atom of each compound, may be a compound in which at least one compound including only one aromatic heterocycle and at least one compound including only one aromatic ring are directly bonded to each other via a single carbon atom of each compound, or may be a fused heterocyclic group, and specific examples of the heterocyclic group include the specific examples of the aromatic heterocyclic groups described above); an ester group; and a thioether group. These groups may optionally be substituted with a halogen atom, a hetero element, an alkyl group, an aromatic ring, or the like.

The dialkylamino group for R^{2a} and R^{2b} preferably has a C1-C20 alkyl group, and more preferably has a C1-C10 alkyl group. The numbers of carbon atoms of the two alkyl groups in the dialkylamino group may be the same as or different from each other. The dialkylamino group is also preferably a cyclic amino group in which two alkyl groups selected from alkyl groups and substituted alkyl groups are linked together.

Specific examples of the dialkylamino group include a dimethylamino group, a diethylamino group, a dipropylamino group, a methylethylamino group, a dibutylamino group, a dipentylamino group, a dihexylamino group, a dioctylamino group, a di(2-ethylhexyl)amino group, a didecylamino group, a didodecylamino group, a dioctadecylamino group, a pyrrolidino group, a piperidino group, a morpholino group, a tetrahydroisoquinolino group, and an isoindolino group.

Examples of an aromatic ring formed by bonding R^{3a} and R^{3b} include C3-C30 aromatic rings including: aromatic hydrocarbon rings such as a benzene ring, a naphthalene ring, an anthracene ring, a phenanthrene ring, and a pyrene ring; nitrogen-containing aromatic rings such as a pyridine ring, a pyrazine ring, a pyrimidine ring, a pyridazine ring, a quinoline ring, an isoquinoline ring, a quinazoline ring, a quinoxaline ring, an acridine ring, a pyrrole ring, an imidazole ring, an indole ring, a benzimidazole ring, and a carbazole ring; oxygen-containing aromatic rings such as a furan ring and a benzofuran ring; and sulfur-containing aromatic rings such as a thiophene ring and a benzothiophene ring.

The aromatic ring formed by bonding R^{3a} and R^{3b} may have one or more monovalent substituents. Examples of the monovalent substituents include the same substituents as those for the aromatic hydrocarbon groups and aromatic heterocyclic groups described above.

In the compound represented by the formula (1), at least one of R^{1a} to R^{1d}, R^{3a}, and R^{3b} is an aromatic hydrocarbon group optionally having a substituent or an aromatic heterocyclic group optionally having a substituent. In other words, at least one of the six, R^{1a} to R^{1d}, R^{3a}, and R^{3b}, is an aromatic hydrocarbon group optionally having a substituent or an aromatic heterocyclic group optionally having a substituent. Preferably, two or more of the six are preferably selected from an aromatic hydrocarbon group optionally having a substituent and an aromatic heterocyclic group optionally having a substituent. When R^{3a} and R^{3b} are bonded to form a fused ring structure, the fused ring structure is counted as one aromatic hydrocarbon group or aromatic heterocyclic group.

When two of R^{1a} to R^{1d}, R^{3a}, and R^{3b} in the compound represented by the formula (1) are selected from an aromatic hydrocarbon group optionally having a substituent and an aromatic heterocyclic group optionally having a substituent, the two preferably correspond to a combination of R^{1a} and R^{1c}, a combination of any two of R^{1b} to R^{1d}, or a combination of R^{3a} and R^{3b}.

When three of R^{1a} to R^{1d}, R^{3a}, and R^{3b} are selected from an aromatic hydrocarbon group optionally having a substituent and an aromatic heterocyclic group optionally having a substituent, preferably, R^{3a} and R^{3b} are bonded to form a fused ring structure, and a combination of R^{1a} and R^{1c} or a combination of any two of R^{1b} to R^{1d} are selected from an aromatic hydrocarbon group optionally having a substituent and an aromatic heterocyclic group optionally having a substituent.

Specific examples of the compound represented by the formula (1) include compounds represented by the following formulas (1-1) to (1-16).

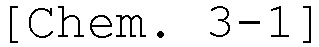

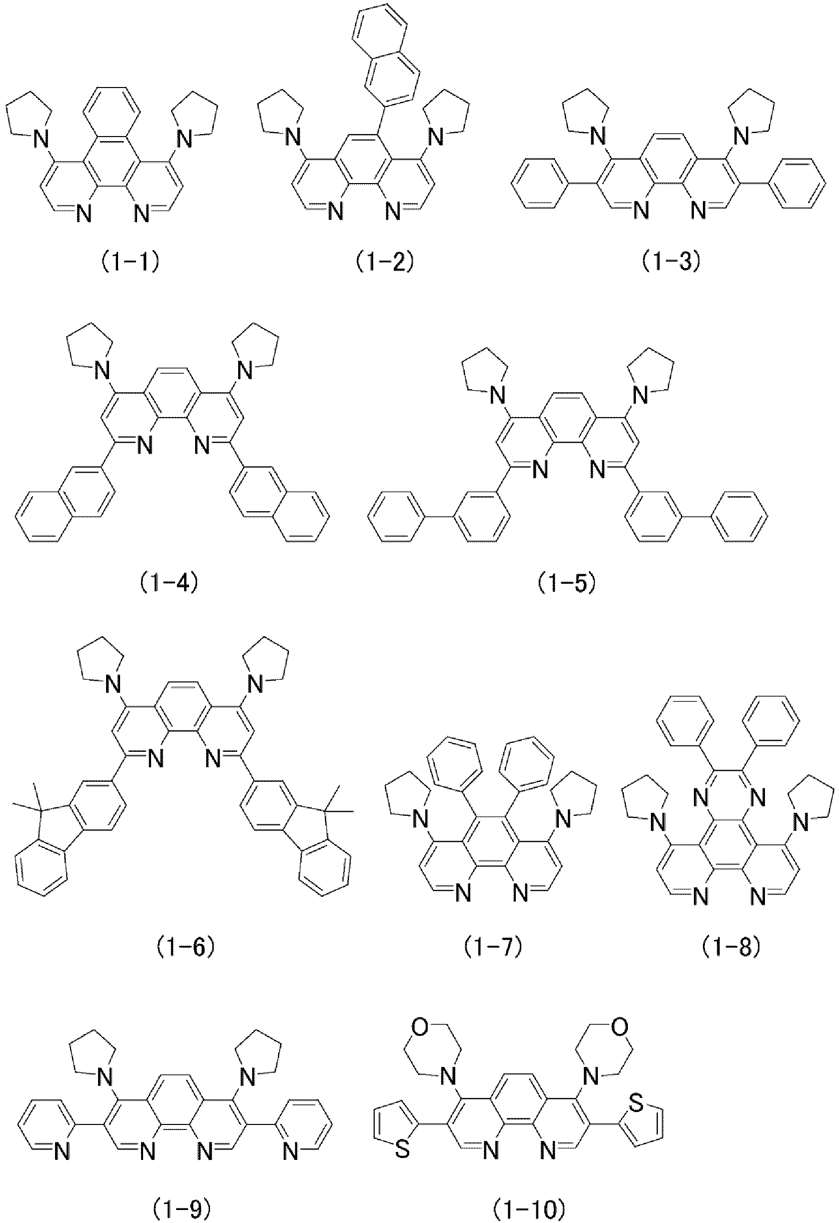

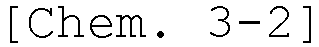

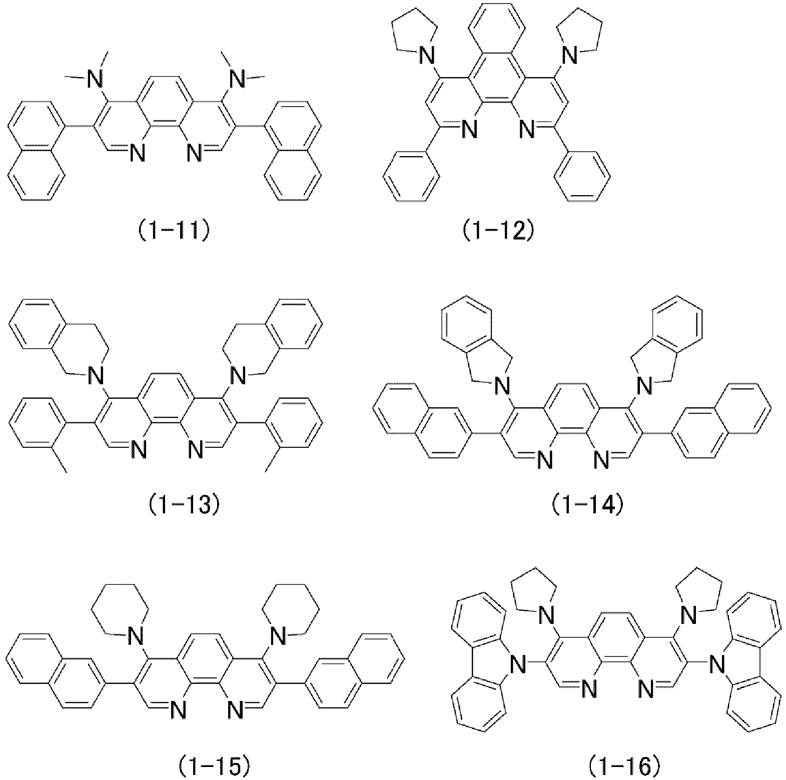

### Organic thin film of the present invention

The organic thin film of the present invention contains a phenanthroline compound represented by the formula (1). The compound of the formula (1) in the organic thin film has a phenanthroline structure, and therefore functions as an n-type dopant that donates electrons to an electron transport material. Furthermore, the compound forms a coordinate bond with the metal of a cathode, changing the work function of the cathode. Thus, the organic thin film of the present invention has good electron injection properties and transport properties.

The organic thin film of the present invention may further contain an electron transport material as a constituent second material. The second material is preferably an organic material. The second material is more preferably an organic material having a lowest unoccupied molecular orbital (LUMO) level of 2.0 eV to 4.0 eV, particularly an n-type organic semiconductor material having a LUMO level of 2.5 eV to 3.5 eV. For example, an electron transport layer of an organic EL device may be made of any of the below-described known materials, and is preferably made of a material that satisfies the above-mentioned LUMO level requirements among the below-described materials.

Specific examples of the second material include phosphine oxide derivatives such as phenyldipyrenylphosphine oxide (POPy2); pyridine derivatives such as tris-1,3,5-(3'-(pyridin-3"-yl)phenyl)benzene (TmPhPyB), 1,3,5-tris(6-(3-(pyridin-3-yl)phenyl)pyridin-2-yl)benzene, and 8,9-diphenyl-7,10-(3-(pyridin-3-yl))fluoranthene; quinoline derivatives such as (2-(3-(9-carbazolyl)phenyl)quinoline (mCQ); pyrimidine derivatives such as 2-phenyl-4,6-bis(3,5-dipyridylphenyl)pyrimidine (BPyPPM), 2-methyl-4,6-bis(3,5-dipyridylphenyl)pyrimidine, and 9-(4-(4,6-diphenylpyrimidin-2-yl)phenyl)-9H-carbazole; pyrazine derivatives; phenanthroline derivatives such as bathophenanthroline (BPhen) and 2,9-dimethyl-4,7-diphenyl-1,10-phenanthroline (BCP); triazine derivatives such as 2,4-bis(4-biphenyl)-6-(4'-(2-pyridinyl)-4-biphenyl)-[1,3,5]triazine (MPT), tris-1,3,5-(3'-(pyridin-3"-yl)phenyl)triazine (TmPhPyTz), tris-1,3,5-([1,1'-biphenyl]-3-yl)triazine, 2-(3-(4,6-di(pyridin-3-yl)-1,3,5-triazin-2-yl)phenyl)-1-phenyl-1H-benzo[d]imidazole, 9-(4-(4,6-diphenyl-1,3,5-triazin-2-yl)phenyl)-9H-3,9'-bicarbazole, 9-(4-(4,6-diphenyl-1,3,5-triazin-2-yl)phenyl)-9H-carbazole, 11-(4,6-diphenyl-1,3,5-triazin-2-yl)-12-phenyl-11,12-dihydroindolo[2,3-a]carbazole, 12-(2-(4,6-diphenyl-1,3,5-triazin-2-yl)phenyl)-12H-benzofuro[2,3,a]-carbazole, and 9-((4-(4,6-dipyridin-3-yl)-1,3,5-triazin-2-yl)phenyl)-9H-carbazole; triazole derivatives such as 3-phenyl-4-(1'-naphthyl)-5-phenyl-1,2,4-triazole (TAZ); oxazole derivatives; oxadiazole derivatives such as 2-(4-biphenylyl)-5-(4-tert-butylphenyl-1,3,4-oxadiazole) (PBD); imidazole derivatives such as 2,2',2"-(1,3,5-bentriyl)-tris(1-phenyl-1-H-benzimidazole) (TPBI); aromatic carboxylic acid anhydrides such as naphthalene-1,4,5,8-tetracarboxylic dianhydride and 3,4,9,10-perylenetetracarboxylic dianhydride; aromatic imide compounds such as N,N'-dimethyl-3,4,9,10-perylenetetracarboxylic diimide; carbonyl group-containing compounds such as isoindigo derivatives, 2,5-dihydropyrrolo[3,4-c]pyrrole-1,4-dione derivatives (diketopyrrolopyrroles), and toluxenone; 1,2,5-thiadiazole derivatives such as naphtho[1,2-c:5,6-c']bis[1,2,5]thiadiazole and benzo[c][1,2,5]thiadiazole; a compound containing a carbonyl-containing heterocycle such as a compound of the below-described formula (30); metal complexes typified by bis[2-(2-hydroxyphenyl)benzothiazolato]zinc (Zn(BTZ)₂) and tris(8-hydroxyquinolinato) aluminum (Alq₃); organic silane derivatives typified by silole derivatives such as 2,5-bis(6'-(2',2"-bipyridyl))-1,1-dimethyl-3,4-diphenylsilole (PyPySPyPy); and boron-containing compounds such as tris(2,4,6-trimethyl-3-(pyridin-3-yl)phenyl)borane (3TPYMB) and compounds disclosed in JP 2013-23961 A, JP 2016-199507 A, JP 2016-199508 A, and WO 2014/133141. One or two or more of these may be used. The second material may also be any of the below-described materials for a light-emitting layer.

More preferred of these second materials are phosphine oxide derivatives such as POPy₂, metal complexes such as Alq₃, pyridine derivatives such as TmPhPyB, and triazine derivatives such as TmPhPyTz.

The second material may further contain a compound not having in its molecule a structure capable of coordinating to an alkali metal. The second material is preferably a compound having in its structure an aromatic heterocycle that does not have a carbon-nitrogen double bond or a compound not having in its molecule a π-electron deficient aromatic heterocycle. The second material is also preferably a compound represented by the following formula (2): 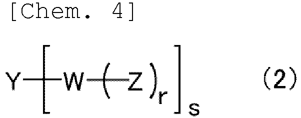 wherein Y represents any one of an aromatic hydrocarbon group, a π-electron-rich aromatic heterocyclic group, or a π-conjugated cyclic structural group containing silicon and/or boron as a constituent element, with each group optionally having a substituent; W represents a linking group or a direct bond; Z represents an aryl group not containing a carbon-nitrogen double bond in its structure; r is an integer of 1 to 6; and s is an integer of 1 to 6.

In the formula (2), Y represents any one of an aromatic hydrocarbon group, a π-electron-rich aromatic heterocyclic group, or a π-conjugated cyclic structural group containing silicon and/or boron as a constituent element, with the groups optionally having a substituent.

Examples of the aromatic hydrocarbon group include C6-C30 aromatic hydrocarbon groups such as a benzene ring, a naphthalene ring, an anthracene ring, a biphenyl ring, a triphenylene ring, a fluoranthene ring, a fluorene ring, a chrysene ring, and a dibenzochrysene ring.

Examples of the π-electron-rich aromatic heterocyclic group include π-electron-rich C3-C20 aromatic heterocyclic groups, such as a thiophene ring, a furan ring, a pyrrole ring, a dibenzothiophene ring, a dibenzofuran ring, and a carbazole ring.

Examples of the π-conjugated cyclic structural group containing silicon and/or boron as a constituent element include C3-C30 groups such as a silole ring, a cyclic structure formed by nitrogen/boron substitution of part of a dibenzo[g,p]chrysene ring, and a cyclic structure formed by nitrogen/boron substitution of part of a spiro-bifluorene ring.

The aromatic hydrocarbon group, the π-electron-rich aromatic heterocyclic group, and the π-conjugated cyclic structural group containing silicon and/or boron as a constituent element may optionally have a substituent. Examples of the substituent include the same groups as the monovalent substituents for R¹ to R³, R⁶, and R⁸ in the formula (1).

In the formula (2), W represents a linking group or a direct bond. When W is a linking group, W is a linking group having a valency of (r + 1).

Examples of the linking group include an arylene group, a heteroarylene group, an ethenylene group, an ethynylene group, a nitrogen atom, an oxygen atom, a sulfur atom, a silicon atom, and a boron atom. These linking groups may optionally have a substituent, and examples of the substituent include the same groups as the monovalent substituents for R¹ to R³, R⁶, and R⁸ in the formula (1).

In the formula (2), Z represents an aryl group not containing a carbon-nitrogen double bond in it structure. Examples of the aryl group not containing a carbon-nitrogen double bond in its structure include C6-C30 groups such as a phenyl group, a tolyl group, a naphthyl group, a triphenylenyl group, a fluoranthenyl group, a dibenzothiophenyl group, a dibenzofuranyl group, and a carbazolyl group. The aryl group may have a substituent as long as it does not contain a carbon-nitrogen double bond in its structure. Examples of the substituent include the same groups as the monovalent substituents for R¹ to R³, R⁶, and R⁸ in the formula (1).

In the formula (2), r is an integer of 1 to 6, and s is an integer of 1 to 6. When r is an integer of 2 to 6, the multiple Zs may be the same as or different from each other. When s is an integer of 2 to 6, the multiple Ws may be the same as or different from each other, and the multiple Zs may be the same as or different from each other.

In the organic thin film of the present invention, electrons smoothly transfer between the cathode and the organic layer due to the coordination reaction of the phenanthroline compound represented by the formula (1) with the metal. Thus, in the present invention, the second material does not need to be a material capable of injecting electrons through interaction with an alkali metal like the material having been conventionally used between the light-emitting layer or the photoelectric conversion layer and the cathode in organic electroluminescent devices and organic thin-film solar cells.

The organic thin film of the present invention may contain a phenanthroline compound and a second material in any ratio. The ratio can be appropriately determined depending on the types of compounds used as the phenanthroline compound and the second material.

In the present invention, the organic thin film containing a second material may be a single film containing a phenanthroline compound and the second material, or may be a stack including a film containing at least a phenanthroline compound and a film containing at least a second material.

The stack may be a stack including a film containing only a phenanthroline compound and a film containing only a second material or may be a stack including a film containing a phenanthroline compound and a second material and a film containing either a phenanthroline compound or a second material.

In the stack including a film containing a phenanthroline compound and a second material and a film containing either a phenanthroline compound or a second material, the film containing either a phenanthroline compound or a second material may be either a film containing a phenanthroline compound or a film containing a second material and is preferably a film containing a second material.

When the organic electroluminescent device includes any of such organic thin films, either the film containing a phenanthroline compound and a second material or the film containing either a phenanthroline compound or a second material may face the cathode. Preferably, the film containing a phenanthroline compound faces the cathode.

When the organic thin film of the present invention is a stack including a film containing a phenanthroline compound and a second material and a film containing only a first material, the two films in the stack each contain a phenanthroline compound. The two films may contain the same phenanthroline compound or different phenanthroline compounds.

Similarly, when the organic thin film of the present invention is a stack including a film containing a phenanthroline compound and a second material and a film containing only a second material, the two films in the stack each contain a second material. The two films may contain the same second material or different second materials.

### Method for producing organic thin film of the present invention

Next, a method for producing the organic thin film of the present invention is described with reference to examples.

The organic thin film of the present invention contains an electron-donating phenanthroline compound having a structure represented by the formula (1). The organic thin film of the present invention can be formed not only by application but also by vapor deposition. Therefore, an organic electroluminescent device including the organic thin film of the present invention can be produced with few process restrictions, and the organic thin film can be easily used as a material for layers constituting an organic electroluminescent device.

When the organic thin film containing a second material is produced by vapor deposition, it can be produced by the same method as that used to produce other layers of the organic electroluminescent device by vapor deposition. For example, a phenanthroline compound and a second material may be vapor-deposited simultaneously or sequentially. When they are vapor-deposited sequentially, either the phenanthroline compound or the second material may be vapor-deposited first. Alternatively, either of them may be vapor-deposited first and then both may be vapor co-deposited, or both may be vapor co-deposited and then either of them may be vapor-deposited. Preferred embodiments of the method for producing the organic thin film of the present invention include such a method for producing an organic thin film including simultaneously vapor-depositing a phenanthroline compound having a structure represented by the formula (1) and a compound not having in its molecule a structure capable of coordinating to an alkali metal as a second material on a surface on which the organic thin film is to be formed.

Preferred embodiments of the method for producing the organic thin film of the present invention also include: a method for producing an organic thin film including first vapor-depositing either a phenanthroline compound or a second material on a surface on which the organic thin film is to be formed, and then vapor-depositing the other or both of the materials; and a method for producing an organic thin film including simultaneously vapor-depositing a phenanthroline compound and a second material on a surface on which the organic thin film is to be formed, and then vapor-depositing either the phenanthroline compound or the second material on the surface on which the organic thin film is to be formed.

The organic thin film of the present invention can also be produced by application. In this case, a coating composition containing a phenanthroline compound is prepared, and the coating composition is applied to produce the organic thin film. The organic thin film containing a second material may be produced as follows: a coating composition containing a phenanthroline compound and a second material may be prepared and applied, or a coating composition containing a phenanthroline compound and a coating composition containing a second material may be prepared separately and applied sequentially. When applied sequentially, either the coating composition containing a phenanthroline compound or the coating composition containing the second material may be applied first. Alternatively, a coating composition containing either of these materials may be applied, and then a coating composition containing both of these materials may be applied, or a coating composition containing both of these materials may be applied, and then a coating composition containing either of these materials may be applied.

Preferred embodiments of the method for producing the organic thin film of the present invention include such a method for producing an organic thin film including applying a coating composition containing a phenanthroline compound having a structure represented by the formula (1) and a compound not having in its molecule a structure capable of coordinating to an alkali metal as a second material on a surface on which the organic thin film is to be formed.

Preferred embodiments of the method for producing the organic thin film of the present invention also include: a method for producing an organic thin film including first applying either a coating composition containing only a phenanthroline compound or a coating composition containing only a second material to a surface on which the organic thin film is to be formed to form a coat, and then applying the other coating composition or a coating composition containing both of the materials to the coat; and a method for producing an organic thin film including applying a coating composition containing both a phenanthroline compound and a second material to form a coat, and applying a coating composition containing either the phenanthroline compound or the second material to the coat.

The following describes a method for producing an organic thin film including preparing a coating composition containing a phenanthroline compound having a structure represented by the formula (1) and a compound not having in its molecule a structure capable of coordinating to an alkali metal as a second material, and applying the coating composition.

The coating compositions can be obtained, for example, by a method including feeding a predetermined amount of a phenanthroline compound and/or a second material to a solvent in a container, and stirring the contents for dissolution.

Examples of the solvent used to dissolve the phenanthroline compound and the second material include an inorganic solvent, an organic solvent, and a solvent mixture containing any of these.

Examples of the inorganic solvent include nitric acid, sulfuric acid, ammonia, hydrogen peroxide, water, and carbon disulfide.

Examples of the organic solvent include ketone solvents such as methyl ethyl ketone (MEK), acetone, diethyl ketone, methyl isobutyl ketone (MIBK), methyl isopropyl ketone (MIPK), diisobutyl ketone, 3,5,5-trimethylcyclohexanone, diacetone alcohol, cyclopentanone, and cyclohexanone; alcohol solvents such as methanol, ethanol, isopropanol, ethylene glycol, diethylene glycol (DEG), and glycerol; ether solvents such as diethyl ether, diisopropyl ether, 1,2-dimethoxyethane (DME), 1,4-dioxane, tetrahydrofuran (THF), tetrahydropyran (THP), anisole, diethylene glycol dimethyl ether (diglyme), and diethylene glycol ethyl ether (carbitol); cellosolve solvents such as methyl cellosolve, ethyl cellosolve, and phenyl cellosolve; aliphatic hydrocarbon solvents such as hexane, pentane, heptane, and cyclohexane; aromatic hydrocarbon solvents such as toluene, xylene, and benzene; aromatic heterocyclic compound solvents such as pyridine, pyrazine, furan, pyrrole, thiophene, and methylpyrrolidone; amide solvents such as N,N-dimethylformamide (DMF) and N,N-dimethylacetamide (DMA); halogen compound solvents such as chlorobenzene, dichloromethane, chloroform, 1,2-dichloroethane, and carbon tetrachloride; ester solvents such as ethyl acetate, methyl acetate, ethyl formate, and ethylene carbonate; sulfur compound solvents such as dimethyl sulfoxide (DMSO) and sulfolane; nitrile solvents such as acetonitrile, propionitrile, and acrylonitrile; and organic acid solvents such as formic acid, acetic acid, trichloroacetic acid, and trifluoroacetic acid. Of these, ketone solvents such as methyl ethyl ketone (MEK), acetone, diethyl ketone, methyl isobutyl ketone (MIBK), methyl isopropyl ketone (MIPK), diisobutyl ketone, 3,5,5-trimethylcyclohexanone, diacetone alcohol, and cyclopentanone are preferred.

Examples of a method for applying the coating composition containing a phenanthroline compound and a second material include various application methods such as spin coating, casting, microgravure coating, gravure coating, bar coating, roll coating, wire bar coating, dip coating, spray coating, screen printing, flexographic printing, offset printing, and inkjet printing.

After the coating composition is applied by any of these methods, annealing is preferably performed. The annealing is preferably performed at 70°C to 200°C for 0.1 to 5 hours in a nitrogen atmosphere or in the air. Such annealing can vaporize the solvent, forming an organic thin film.

### Organic electroluminescent device of the present invention

The present invention also relates to an organic electroluminescent device (organic EL device) including: a light-emitting layer between a cathode and an anode; and any one of a layer of the organic thin film of the present invention, a layer of a stack including a layer of the organic thin film and a metal oxide layer, or a layer containing the organic electroluminescent device material of the present invention.

In the organic electroluminescent device of the present invention, the layer of the organic thin film, the layer of a stack including a layer of the organic thin film and a metal oxide layer, and the layer containing the organic electroluminescent device material of the present invention may be disposed between the cathode and the light-emitting layer or between the anode and the light-emitting layer, and are preferably disposed between the cathode and the light-emitting layer.

The layer containing the organic electroluminescent device material of the present invention may contain the second material or a different component as long as it contains a phenanthroline compound represented by the formula (1). Preferably, the layer containing the organic electroluminescent device material of the present invention is made of only a phenanthroline compound represented by the formula (1).

Next, the organic electroluminescent device of the present invention is described in detail with reference to examples.

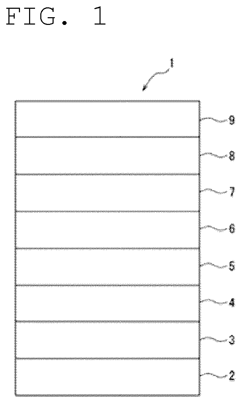

FIG. 1 is a schematic cross-sectional view illustrating an example of the organic electroluminescent device of the present invention. An organic electroluminescent device 1 of the present embodiment shown in FIG. 1 includes a light-emitting layer 6 between an anode 3 and a cathode 9. The organic electroluminescent device 1 shown in FIG. 1 includes an electron injection layer 8 between the cathode 9 and the light-emitting layer 6, with the electron injection layer 8 being made of the organic thin film of the present invention or the organic electroluminescent device material of the present invention.

The organic electroluminescent device 1 of the present embodiment has a stack structure including, on a substrate 2, the anode 3, a hole injection layer 4, a hole transport layer 5, the light-emitting layer 6, an electron transport layer 7, the electron injection layer 8, and the cathode 9, in this order.

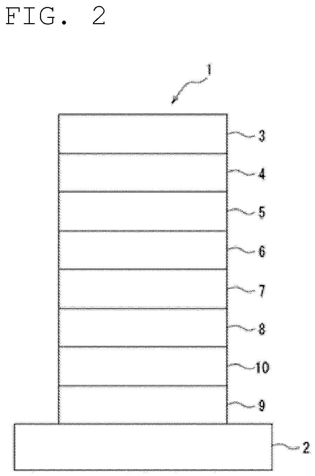

The organic electroluminescent device 1 shown in FIG. 1 is a typical organic electroluminescent device in which the anode 3 is on the side closer to the substrate 2. Preferred embodiments of the organic electroluminescent device of the present invention also include the organic electroluminescent device 1 having an inverted structure in which the cathode 9 is disposed between the substrate 2 and the light-emitting layer 6, as shown in FIG. 2.

Preferred embodiments of the organic electroluminescent device of the present invention also include one having a structure in which an inorganic oxide layer 10 is disposed between the cathode 9 and the electron injection layer 8 made of the organic thin film of the present invention or the organic electroluminescent device material of the present invention.

The organic electroluminescent device 1 shown in FIGS. 1 and 2 may be a top emission device in which light is extracted from the side opposite to the substrate 2, or may be a bottom emission device in which light is extracted from a substrate 2 side.

### <Substrate>

Examples of a material for the substrate 2 include resin materials and glass materials.

Examples of the resin materials for the substrate 2 include polyethylene terephthalate, polyethylene naphthalate, polypropylene, cycloolefin polymers, polyamide, polyethersulfone, polymethyl methacrylate, polycarbonate, and polyarylate. The substrate 2 is preferably made of any of the resin materials because the organic electroluminescent device 1 can have excellent flexibility.

Examples of the glass materials for the substrate 2 include quartz glass and soda glass.

When the organic electroluminescent device 1 is a bottom emission device, the substrate 2 is made of a transparent substrate.

When the organic electroluminescent device 1 is a top emission device, the substrate 2 may be made of a transparent substrate or may be made of an opaque substrate. Examples of the opaque substrate include substrates made of ceramic materials such as alumina, substrates prepared by forming an oxide film (insulating film) on a surface of a metal plate such as a stainless steel plate, and substrates made of resin materials.

The average thickness of the substrate 2 can be determined depending on, for example, the material of the substrate 2 and is preferably 0.02 to 30 mm, more preferably 0.05 to 10 mm.

The average thickness of the substrate 2 can be measured using a digital multimeter or a vernier caliper.

### <Anode>

The anode 3 shown in FIG. 1 is formed on and in direct contact with the substrate 2. The anode 3 does not have to be formed on and in direct contact with the substrate 2 in the inverted organic electroluminescent device shown in FIG. 2.

Examples of a material for the anode 3 include conductive oxide materials such as indium tin oxide (ITO), indium zinc oxide (IZO), fluorine tin oxide (FTO), In₃O₃, SnO₂, Sb-containing SnO₂, and Al-containing ZnO. Of these materials for the anode 3, ITO, IZO, or FTO is preferably used.

The average thickness of the anode 3 is preferably, but not limited to, 10 to 500 nm, more preferably 100 to 200 nm.

The average thickness of the anode 3 can be measured with a stylus profiler or by spectroscopic ellipsometry.

### <Hole injection layer>

The hole injection layer 4 may be made of an inorganic material or an organic material.

Non-limiting examples of the inorganic material include metal oxides such as vanadium oxide (V₂O₅), molybdenum oxide (MoO₃), and ruthenium oxide (RuO₂). These may be used alone or in combination of two or more of these.

Examples of the organic material that can be used include low-molecular-weight materials such as dipyrazino[2,3-f:2',3'-h]quinoxaline-2,3,6,7,10,11-hexacarbonitrile (HAT-CN) and 2,3,5,6-tetrafluoro-7,7,8,8-tetracyano-quinodimethane (F4-TCNQ), and poly(3,4-ethylenedioxythiophene)/poly(styrene sulfonate) (PEDOT/PSS).

The average thickness of the hole injection layer 4 is preferably, but not limited to, 1 to 1000 nm, more preferably 5 to 50 nm.

The average thickness of the hole injection layer 4 can be measured with a stylus profiler or by spectroscopic ellipsometry.

### <Hole transport layer>

Examples of a hole transport organic material for the hole transport layer 5 include p-type high-molecular-weight materials (organic polymers) and p-type low-molecular-weight materials. These materials may be used alone or in combination.

Specific examples of the material for the hole transport layer 5 include N,N'-di(1-naphthyl)-N,N'-diphenyl-1,1'-biphenyl-4,4'-diamine (α-NPD), N4,N4'-bis(dibenzo[b,d]thiophen-4-yl)-N4,N4'-diphenylbiphenyl-4,4'-diamine (DBTPB), polyarylamine, fluorene-arylamine copolymers, fluorene-bithiophene copolymers, poly(N-vinylcarbazole), polyvinylpyrene, polyvinylanthracene, polythiophene, polyalkylthiophenes, polyhexylthiophene, poly(p-phenylenevinylene), polytinylenevinylene, pyrene formaldehyde resin, ethylcarbazole formaldehyde resin, and derivatives thereof. These materials for the hole transport layer 5 can also be used as a mixture with a different compound. An example of a mixture containing polythiophene as the material for the hole transport layer 5 is poly(3,4-ethylenedioxythiophene)/poly(styrene sulfonate) (PEDOT/PSS).

The average thickness of the hole transport layer 5 is preferably, but not limited to, 10 to 150 nm, more preferably 20 to 100 nm.

The average thickness of the hole transport layer 5 can be measured with a stylus profiler or by spectroscopic ellipsometry.

### <Light-emitting layer>

The light-emitting layer 6 may be made of any of materials that can be commonly used for the light-emitting layer 6 or may be made of a mixture of these. Specifically, for example, the light-emitting layer 6 may contain bis[2-(2-benzothiazolyl)phenolato]zinc(II) (Zn(BTZ)₂) and tris[1-phenylisoquinoline]iridium(III) (Ir(piq)₃).

The light-emitting layer 6 may be made of a low-molecular-weight compound or a high-molecular-weight compound. The term "low-molecular-weight material" as used herein refers to a material that is not a high-molecular-weight material (polymer), and does not necessarily refer to a low-molecular-weight organic compound.

Examples of the high-molecular-weight material for the light-emitting layer 6 include polyacetylene compounds such as trans-polyacetylene, cis-polyacetylene, poly(diphenylacetylene) (PDPA), and poly(alkylphenylacetylene) (PAPA); polyparaphenylenevinylene compounds such as poly(para-phenylenevinylene) (PPV), poly(2,5-dialkoxy-para-phenylenevinylene) (RO-PPV), cyano-substituted-poly(para-phenylenevinylene) (CN-PPV), poly(2-dimethyloctylsilyl-para-phenylenevinylene) (DMOS-PPV), and poly(2-methoxy-5-(2'-ethylhexoxy)-para-phenylenevinylene) (MEH-PPV); polythiophene compounds such as poly(3-alkylthiophene) (PAT) and poly(oxypropylene)triol (POPT); polyfluorene compounds such as poly(9,9-dialkylfluorene) (PDAF), poly(dioctylfluorene-alt-benzothiadiazole) (F8BT), α,ω-bis[N,N'-di(methylphenyl)aminophenyl]-poly[9,9-bis(2-ethylhexyl)fluorene-2,7-diyl] (PF2/6am4), and poly(9,9-dioctyl-2,7-divinylenefluorenyl-ortho-co(anthracene-9,10-diyl); polyparaphenylene compounds such as poly(para-phenylene) (PPP) and poly(1,5-dialkoxy-para-phenylene) (RO-PPP); polycarbazole compounds such as poly(N-vinylcarbazole) (PVK); polysilane compounds such as poly(methylphenylsilane) (PMPS), poly(naphthylphenylsilane) (PNPS), and poly(biphenylylphenylsilane) (PBPS); and boron compound high-molecular-weight materials disclosed in JP 2011-184430 A and JP 2012-152248 A.

Examples of the low-molecular-weight material for the light-emitting layer 6 include various metal complexes such as a tridentate iridium complex having 2,2'-bipyridine-4,4'-dicarboxylic acid as a ligand, tris(2-phenylpyridine)iridium (Ir(ppy)₃), 8-hydroxyquinoline aluminum (Alq₃), tris(4-methyl-8-quinolinolato)aluminum(III) (Almq₃), 8-hydroxyquinoline zinc (Znq₂), (1,10-phenanthroline)-tris-(4,4,4-trifluoro-1-(2-thienyl)-butane-1,3-dionate)europium(III) (Eu(TTA)₃(phen)), and 2,3,7,8,12,13,17,18-octaethyl-21H,23H-porphine platinum (II); benzene compounds such as distyrylbenzene (DSB) and diaminodistyrylbenzene (DADSB); naphthalene compounds such as naphthalene and Nile Red; phenanthrene compounds such as phenanthrene; chrysene compounds such as chrysene and 6-nitrochrysene; perylene compounds such as perylene and N,N'-bis(2,5-di-t-butylphenyl)-3,4,9,10-perylene-di-carboximide (BPPC); coronene compounds such as coronene; anthracene compounds such as anthracene and bisstyrylanthracene; pyrene compounds such as pyrene; pyran compounds such as 4-(dicyanomethylene)-2-methyl-6-(para-dimethylaminostyryl)-4H-pyran (DCM); acridine compounds such as acridine; stilbene compounds such as stilbene; thiophene compounds such as 2,5-dibenzoxazolethiophene; benzoxazole compounds such as benzoxazole; benzimidazole compounds such as benzimidazole; benzothiazole compounds such as 2,2'-(para-phenylenedivinylene)-bisbenzothiazole; butadiene compounds such as bistyryl(1,4-diphenyl-1,3-butadiene) and tetraphenylbutadiene; naphthalimide compounds such as naphthalimide; coumarin compounds such as coumarin; perynone compounds such as perynone; oxadiazole compounds such as oxadiazole; aldazine compounds; cyclopentadiene compounds such as 1,2,3,4,5-pentaphenyl-1,3-cyclopentadiene (PPCP); quinacridone compounds such as quinacridone and quinacridone red; pyridine compounds such as pyrrolopyridine and thiadiazolopyridine; spiro compounds such as 2,2',7,7'-tetraphenyl-9,9'-spirobifluorene; metallic or non-metallic phthalocyanine compounds such as phthalocyanine (H₂Pc) and copper phthalocyanine; and boron compound materials disclosed in JP 2009-155325 A, JP 2011-184430 A, and JP 2012-151149.

The average thickness of the light-emitting layer 6 is preferably, but not limited to, 10 to 150 nm, more preferably 20 to 100 nm.

The average thickness of the light-emitting layer 6 may be measured with a stylus profiler, or may be measured during formation of the light-emitting layer 6 with a quartz crystal film thickness monitor.

### <Electron transport layer>

The electron transport layer 7 may include any of materials that have conventionally been used as materials for electron transport layers and are used as the second material in the electron injection layer 8.

Although the number of materials needed to produce an organic electroluminescent device increases, a compound capable of injecting electrons from an alkali metal and having been conventionally used as a material for the electron transport layer may be used as a hole blocking layer between the light-emitting layer 6 and the electron transport layer 7, for example.

The average thickness of the electron transport layer 7 is preferably, but not limited to, 10 to 150 nm, more preferably 20 to 100 nm.

The average thickness of the electron transport layer 7 can be measured with a stylus profiler or by spectroscopic ellipsometry.

As described later, the electron transport layer may be omitted when the organic thin film made of a mixture of the phenanthroline compound of the present invention and a second material serves as the electron injection layer 8 and the electron transport layer 7.

### <Electron injection layer>

The electron injection layer 8 serves to improve the speed of electron injection from the cathode 9 to the light-emitting layer 6 and the electron transport properties. The electron injection layer 8 is made of an organic thin film containing the phenanthroline compound of the present invention.

The average thickness of the electron injection layer 8 is preferably 0.5 to 100 nm, more preferably 1 to 50 nm.

The electron injection layer 8 made of the organic thin film containing the phenanthroline compound of the present invention can be obtained by application or vacuum deposition of a coating composition containing the phenanthroline compound of the present invention. When the average thickness of the electron injection layer 8 is 100 nm or less, an increase in the driving voltage of the organic electroluminescent device 1 caused by providing the electron injection layer 8 can be sufficiently prevented.

When the electron injection layer 8 is a film containing the phenanthroline compound of the present invention and a second material, any of the following structures may be used.

Examples include a structure in which a film of a mixture of the phenanthroline compound of the present invention and a second material is the electron injection layer 8 and a film made of a second material is the electron transport layer 7; a structure in which a film consisting of the phenanthroline compound of the present invention is the electron injection layer 8 and a film consisting of a second material is the electron transport layer 7; a structure in which a stack including a film consisting of a second material and a film consisting of the phenanthroline compound of the present invention is the electron injection layer 8 and a film consisting of a second material is the electron transport layer 7; a structure in which a stack including a film made of a second material and a film of a mixture of the phenanthroline compound of the present invention and a second material is the electron injection layer 8, with the film made of a second material facing the cathode 9, and a film consisting of a second material is the electron transport layer 7; and a structure in which a film of a mixture of the phenanthroline compound of the present invention and a second material serves as the electron injection layer 8 and the electron transport layer 7 described above. When two adjacent films both contain a second material, the two films may contain the same second material or different second materials.

The average thickness of the electron injection layer 8 can be measured with a stylus profiler or by spectroscopic ellipsometry.

It has been reported that when a metal is vapor-deposited on an organic thin film or when an organic thin film is vapor-deposited on a cathode, the metal diffuses toward the organic thin film by a distance of several nanometers (see Lee, J.H. et al., Direct evidence of Al diffusion into tris-(8-hydroquinone) aluminum layer: medium energy ion scattering analysis. Applied Physics Letters 93, doi:10.1063/1.3002290 (2008) and Fukagawa, H. et al., Long-Lived Flexible Displays Employing Efficient and Stable Inverted Organic Light-Emitting Diodes. Adv. Mater. 30, e1706768, doi:10.1002/adma. 201706768 (2018)). Therefore, even in a stack structure in which the second material faces the cathode 9, the coordination reaction of the diffused metal with the phenanthroline compound of the present invention can give the effect of electron injection. In other words, the effect of the present invention can be obtained without forming the phenanthroline compound of the present invention directly on the cathode 9. When the organic electroluminescent device includes a cathode and a layer of the organic thin film of the present invention under the cathode in a stack structure, the organic electroluminescent device can be said to include the stack in the present invention.

The present invention encompasses such an organic electroluminescent device including the stack in the present invention.

The organic electroluminescent device of the present invention may include a stack as the electron injection layer 8. In other words, preferred embodiments of the organic electroluminescent device of the present invention include one including between the cathode 9 and the light-emitting layer 6 a stack including a film containing the phenanthroline compound of the present invention and a second material and a film containing the second material.

In this case, preferred embodiments of the organic electroluminescent device of the present invention include: the organic electroluminescent device including the layer containing a second material between the light-emitting layer 6 and the film containing the phenanthroline compound of the present invention and a second material; and the organic electroluminescent device including the layer containing a second material between the cathode 9 and the film containing a first material and a second material.

### <Cathode>

Examples of a material for the cathode 9 include ITO, IZO, Au, Pt, Ag, Cu, Al, Mg, and alloys containing these. Of these materials for the cathode 9, ITO, IZO, Au, Ag, or Al is preferably used.

The average thickness of the cathode 9 is preferably, but not limited to, 10 to 1000 nm, more preferably 30 to 150 nm. Even when the cathode 9 is made of an opaque material, the cathode 9 having an average thickness of about 10 to 30 nm can be used as a transparent anode for a top emission organic electroluminescent device.

The average thickness of the cathode 9 can be measured during the formation of the cathode 9 with a quartz crystal film thickness monitor.

### <Electron injection oxide layer>

When the organic thin film of the present invention is used in the inverted organic electroluminescent device shown in FIG. 2, the inorganic oxide layer 10 is preferably disposed on the cathode 9. The oxide layer 10 can function as the electron injection layer 8 and/or the cathode 9. The electron injection layer 8 can be used between the light-emitting layer 6 and the cathode 9 in an inverted organic electroluminescent device, but the oxide layer 10 is disposed between the cathode 9 and an organic layer.

The oxide layer 10 is a layer of a semiconductor or insulator thin-film stack. Specifically, the oxide layer 10 may be a layer consisting of a single metal oxide; a stack of layers each consisting of a combination of two or more different metal oxides, a stack of layers each consisting of a single metal oxide, or a stack of a layer(s) each consisting of a combination of two or more different metal oxides and a layer(s) consisting of a single metal oxide; or a layer consisting of a combination of two or more different metal oxides.

Examples of metal element(s) constituting the metal oxide that forms the oxide layer 10 include magnesium, calcium, strontium, barium, titanium, zirconium, hafnium, vanadium, niobium, tantalum, chromium, molybdenum, tungsten, manganese, indium, gallium, iron, cobalt, nickel, copper, zinc, cadmium, aluminum, and silicon.

When the oxide layer 10 includes a layer consisting of a combination of two or more different metal oxides, at least one of the metal elements of the metal oxides in the layer is preferably magnesium, aluminum, calcium, zirconium, hafnium, silicon, titanium, or zinc.

When the oxide layer 10 is a layer consisting of a single metal oxide, the metal oxide in the layer is preferably selected from the group consisting of magnesium oxide, aluminum oxide, zirconium oxide, hafnium oxide, silicon oxide, titanium oxide, and zinc oxide.

When the oxide layer 10 is a stack of layers each consisting of a combination of two or more different metal oxides, a stack of layers each consisting of a single metal oxide, a stack of a layer(s) each consisting of a combination of two or more different metal oxides and a layer(s) consisting of a single metal oxide, or a layer consisting of a combination of two or more different metal oxides, two different metal oxides may be stacked and/or combined selected as a combination from, for example, titanium oxide/zinc oxide, titanium oxide/magnesium oxide, titanium oxide/zirconium oxide, titanium oxide/aluminum oxide, titanium oxide/hafnium oxide, titanium oxide/silicon oxide, zinc oxide/magnesium oxide, zinc oxide/zirconium oxide, zinc oxide/hafnium oxide, zinc oxide/silicon oxide, and calcium oxide/aluminum oxide; and three different metal oxides may be stacked and/or combined selected as a combination from, for example, titanium oxide/zinc oxide/magnesium oxide, titanium oxide/zinc oxide/zirconium oxide, titanium oxide/zinc oxide/aluminum oxide, titanium oxide/zinc oxide/hafnium oxide, titanium oxide/zinc oxide/silicon oxide, and indium oxide/gallium oxide/zinc oxide.

The oxide layer 10 may contain indium gallium zinc oxide (IGZO) which is an oxide semiconductor with a particular composition and good characteristics and/or a 12CaO·7Al₂O₃ electride.

The average thickness of the oxide layer 10 is preferably, but not limited to, 1 to 1000 nm, more preferably 2 to 100 nm.

The average thickness of the oxide layer 10 can be measured with a stylus profiler or by spectroscopic ellipsometry.

### <Sealing>

The organic electroluminescent device 1 shown in FIG. 1 and FIG. 2 may be sealed, as necessary.

For example, the organic electroluminescent device 1 shown in FIG. 1 and FIG. 2 may be sealed using an airtight container (not shown) having a concave space for containing the organic electroluminescent device 1, with the edge of the airtight container being bonded to the substrate 2 with an adhesive. Alternatively, the organic electroluminescent device 1 may be contained in the airtight container and sealed by filling the container with a sealant made of an ultraviolet (UV) curable resin or the like.

Also, for example, the organic electroluminescent device 1 shown in FIG. 1 may be sealed using sealing members including a plate member (not shown) placed on the cathode 9 and a frame member (not shown) placed along a cathode 9-side edge of the plate member, with the plate member being bonded to the frame member and the frame member being bonded to the substrate 2 with an adhesive.

Also, the organic electroluminescent device 1 shown in FIG. 2 may be sealed using sealing members including a plate member (not shown) placed on the anode 3 and a frame member (not shown) placed along an anode 3-side edge of the plate member, with the plate member being bonded to the frame member and the frame member being bonded to the substrate 2 with an adhesive.

When the organic electroluminescent device 1 is sealed using the airtight container or the sealing members, a desiccant to absorb moisture may be placed in the airtight container or within the sealing members. Also, the airtight container or the sealing members may be made of a moisture absorbing material. There may be a space in the airtight container or within the sealing members after sealing.

Examples of a material for the airtight container or the sealing members used to seal the organic electroluminescent device 1 shown in FIG. 1 and FIG. 2 include a resin material and a glass material. Examples of the resin material and glass material for the airtight container or sealing members include those mentioned as the material for the substrate 2.

In the organic electroluminescent device 1 of the present embodiment, when the electron injection layer 8 is formed using an organic thin film made of the phenanthroline compound of the present invention and a compound not having in its molecule a structure capable of coordinating to an alkali metal as a second material, better durability can be achieved compared to, for example, a case where an alkali metal, which is a material unstable in the air, is used in the electron injection layer 8. Therefore, an airtight container or sealing members having a water vapor transmission rate of about 10⁻⁴ to 10⁻³ g/m²/day can sufficiently prevent degradation of the organic electroluminescent device 1. Thus, a resin material having a water vapor transmission rate of higher than about 10⁻⁴ g/m²/day can be used as the material for the airtight container or sealing members, and the organic electroluminescent device 1 can have excellent flexibility.

### Method for producing organic electroluminescent device

Next, as an example of a method for producing the organic electroluminescent device of the present invention, a method for producing the organic electroluminescent device 1 shown in FIG. 1 is described.

To produce the organic electroluminescent device 1 shown in FIG. 1, first, the anode 3 is formed on the substrate 2.

The anode 3 may be formed by, for example, a sputtering method, a vacuum vapor deposition method, a sol-gel method, a spray pyrolysis deposition (SPD) method, an atomic layer deposition (ALD) method, a vapor deposition method, or a liquid phase deposition method. The anode 3 may be formed by bonding metal foil.

Next, the hole injection layer 4 is formed on the anode 3.

The hole injection layer 4 can be formed by the method for producing the organic thin film described above.

Next, on the hole injection layer 4, the hole transport layer 5, the light-emitting layer 6, the electron transport layer 7, and the electron injection layer 8 are formed in this order.

The hole transport layer 5, the emitting layer 6, the electron transport layer 7, and the electron injection layer 8 may be formed by any methods. Appropriate methods selected from known various forming methods can be used depending on the properties of the materials for the hole transport layer 5, the emitting layer 6, the electron transport layer 7, and the electron injection layer 8.

Specifically, the hole transport layer 5, the emitting layer 6, the electron transport layer 7, and the electron injection layer 8 may be formed by, for example, application, vacuum vapor deposition, or evaporative spray deposition from ultra-dilute solution (ESDUS) of organic compound solutions containing organic compounds for the hole transport layer 5, the emitting layer 6, the electron transport layer 7, and the electron injection layer 8.

When the hole transport layer 5, the light-emitting layer 6, the electron transport layer 7, and the electron injection layer 8 are formed by application, the organic compounds for the hole transport layer 5, the light-emitting layer 6, the electron transport layer 7, and the electron injection layer 8 are separately dissolved in solvents to prepare the organic compound solutions containing the organic compounds for the hole transport layer 5, the light-emitting layer 6, the electron transport layer 7, and the electron injection layer 8.

Preferred examples of the solvents for dissolving the organic compounds for the electron transport layer 5, the light-emitting layer 6, the electron transport layer 7, and the electron injection layer 8 include aromatic hydrocarbon solvents such as xylene, toluene, cyclohexylbenzene, dihydrobenzofuran, trimethylbenzene, and tetramethylbenzene; aromatic heterocyclic compound solvents such as pyridine, pyrazine, furan, pyrrole, thiophene, and methylpyrrolidone; and aliphatic hydrocarbon solvents such as hexane, pentane, heptane, and cyclohexane. These can be used alone or in combination.

The organic compound solutions containing the organic compounds for the hole transport layer 5, the light-emitting layer 6, the electron transport layer 7, and the electron injection layer 8 may be applied by various application methods such as spin coating, casting, microgravure coating, gravure coating, bar coating, roll coating, wire bar coating, dip coating, spray coating, screen printing, flexographic printing, offset printing, and inkjet printing. Of these application methods, spin coating and slit coating are preferred in that they allow for easy control of the film thicknesses.

Next, the cathode 9 is formed.

The cathode 9 can be formed, for example, in the same manner as the anode 3.

Through the above steps, the organic electroluminescent device 1 shown in FIG. 1 can be obtained.

### Sealing method

When the organic electroluminescent device 1 shown in FIG. 1 is sealed, it can be sealed by a method commonly used for sealing organic electroluminescent devices.

In the organic electroluminescent device 1 of the present embodiment, the phenanthroline compound forms a coordinate bond with the adjacent cathode material, generating a negative charge, and thus providing excellent electron injection properties. Therefore, the speed of electron injection and electron transport from the cathode 9 to the light-emitting layer 6 is high, resulting in the organic electroluminescent device 1 with a low driving voltage.

As described above, other embodiments of the organic electroluminescent device of the present invention include the organic electroluminescent device 1 in which the organic thin film containing a phenanthroline compound and a second material is a stack in which a layer made of the second material and an electron injection layer made of a first material are separately provided. In the organic electroluminescent device having such a structure, the speed of electron injection and electron transport from the cathode 9 to the light-emitting layer 6 is also high, resulting in the organic electroluminescent device 1 with a low driving voltage.

### Other examples

The organic electroluminescent device of the present invention is not limited to the organic electroluminescent devices described in the aforementioned embodiments.

Specifically, although the cases where the organic thin film functions as an electron injection layer are exemplified in the aforementioned embodiments, the organic electroluminescent device of the present invention may be any one including an organic thin film between the cathode and the light-emitting layer. Thus, the organic thin film is not limited to an electron injection layer, and may be disposed as an electron transport layer or a layer serving both as an electron injection layer and an electron transport layer.

The organic electroluminescent device 1 shown in FIG. 1 may or may not include the electron transport layer 7, the hole transport layer 5, or the hole injection layer 4, as necessary.

Each of the anode 3, the hole injection layer 4, the hole transport layer 5, the light-emitting layer 6, the electron transport layer 7, the electron injection layer 8, and the cathode 9 may be a single layer or may consist of two or more layers.

The organic electroluminescent device 1 shown in FIG. 1 may include a different layer between any of the layers shown in FIG. 1. Specifically, in order to further improve the properties of the organic electroluminescent device, the organic electroluminescent device may include a hole blocking layer or the like as necessary.

As described above, the phenanthroline compound of the present invention has greatly excellent electron injection properties. When the electron injection layer 8 contains such a phenanthroline compound, electrons can be directly injected into the material used in the light-emitting layer 6. Therefore, also when the electron transport layer 7 contains the material used in the light-emitting layer 6, the device can be operated at a low driving voltage. This allows the organic EL device 1 having a simple structure in which the electron transport layer 7 contains the material used in the light-emitting layer 6 to operate at a low driving voltage. Such a device allows for the reduction of one material compared to typical organic EL devices.

Therefore, when the electron injection layer 8 contains the phenanthroline compound of the present invention and the electron transport layer 7 contains a second material, the light-emitting layer 6 can also contain the second material. In other words, preferred embodiments of the organic EL device of the present invention also include an embodiment in which the light-emitting layer 6 contains the second material.

Furthermore, holes can be relatively easily injected from the hole injection layer 4 into the material used in the light-emitting layer 6. Thus, the device in which the electron injection layer 8 contains the phenanthroline compound of the present invention can be operated at a low driving voltage even when the hole transport layer 5 contains the material used in the light-emitting layer 6. Thus, the organic EL device 1 having a simple structure in which the electron transport layer 7 and the hole transport layer 5 contain the material used in the light-emitting layer 6 can be operated at a low driving voltage. Such a device allows for the reduction of two materials compared to typical organic EL devices.

Therefore, when the electron injection layer 8 contains the phenanthroline compound of the present invention and the electron transport layer 7 contains a second material, the light-emitting layer 6 and the hole transport layer 5 can also contain a second material. In other words, preferred embodiments of the organic EL device of the present invention also include an embodiment in which the light-emitting layer 6 contains the second material. Preferred embodiments of the organic EL device of the present invention also include an embodiment in which a layer containing the second material (for example, the hole transport layer 5) is disposed between the anode 3 and the light-emitting layer 6.

In the aforementioned embodiments, the conventional organic EL device including the anode 3, the light-emitting layer 6, and the cathode 9 in this order on the substrate 2 is exemplified. The organic EL device of the present invention may however be an inverted organic EL device including the cathode 9 between the substrate 2 and the light-emitting layer 6 (see, for example, FIG. 2).

The organic EL device shown in FIG. 2 may or may not include the inorganic oxide layer 10, the electron transport layer 7, the hole transport layer 5, or the hole injection layer 4 as necessary.

### Display, lighting system

The organic electroluminescent device of the present invention is capable of varying the color of light by suitably selecting the material for the light-emitting layer, etc., and capable of providing a desired color of light by using a color filter, etc. together therewith. Thus, the organic electroluminescent device of the present invention can be suitably used as a light-emitting portion of a display or in a lighting system.

The display of the present invention includes the organic electroluminescent device of the present invention that includes an organic thin film between the cathode and the light-emitting layer, that can be produced at a high yield, and that has a low driving voltage. Thus, the display of the present invention is suitable as a display.

The lighting system of the present invention includes the organic electroluminescent device of the present invention that can be produced at a high yield and that has a low driving voltage. Thus, the lighting system is suitable as a lighting system.

### Organic thin-film solar cell, thin-film transistor

The present invention is not limited to those of the above-described embodiments, and the organic thin film of the present invention can be used, for example, in devices such as organic thin-film solar cells and thin-film transistors.

The organic thin-film solar cell of the present invention includes the organic thin film of the present invention. For example, when the organic thin film is used in the electron injection layer of an organic thin-film solar cell, hydrogen bonding is formed between the phenanthroline compound of the present invention and a second material so that a negative charge generates. Thereby, electrons can be transported at a high speed and high power generation efficiency can be obtained. Thus, the organic thin-film solar cell of the present invention is suitable as an organic thin-film solar cell.

The thin-film transistor of the present invention includes an organic thin film. For example, when the channel layer of the thin-film transistor is formed from the organic thin film, the channel layer can have high electron mobility.

When the organic thin film is formed on an electrode, a reduction in contact resistance can be expected.

### EXAMPLES

The present invention is described in more detail below with reference to examples, but the present invention is not limited to these examples. The terms "part(s)" and "%" refer to "part(s) by mass" and "% by mass", respectively, unless otherwise stated.

### (Example 1) Synthesis of compound (1-1)

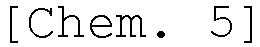

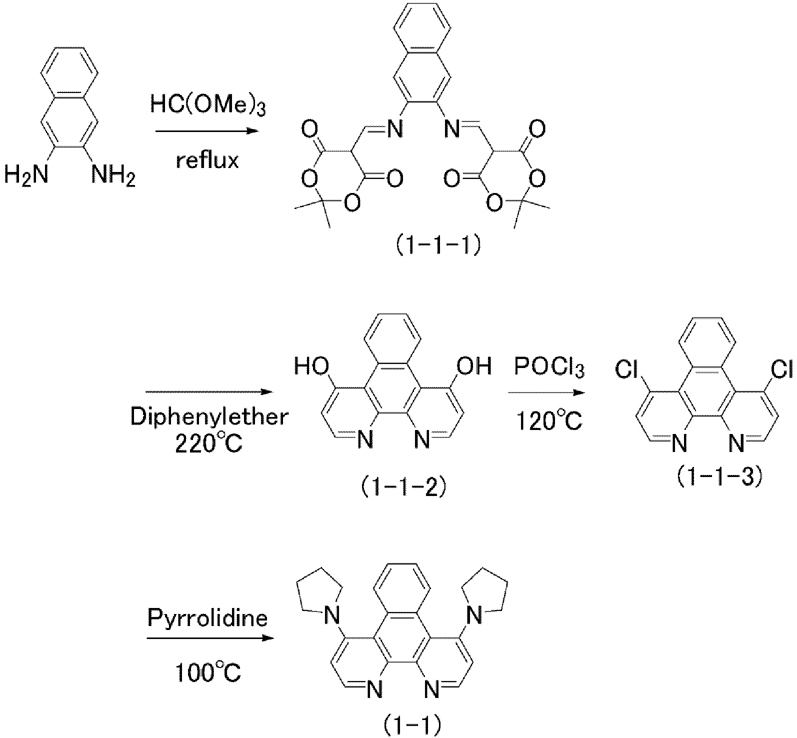

A flask was charged with 10.4 g of Meldrum's acid and 90 mL of methyl orthoformate, and the contents were heated and stirred at 130°C for two hours. The contents were cooled to 70°C, 5.0 g of 1,2-diaminonaphthalene was added thereto, and the contents were heated and stirred at 130°C for two hours. After the contents were cooled to room temperature, the precipitated solid was collected by filtration, rinsed with diethyl ether, and dried under vacuum to obtain 12.5 g of a compound (1-1-1). The compound (1-1-1) was gradually added to diphenyl ether heated to 220°C, and the contents were heated and stirred for 30 minutes. After the contents were cooled to room temperature, acetone was added thereto, and the precipitated solid was collected by filtration and rinsed with acetone to obtain 3.8 g of a brown solid (1-1-2).

A flask was charged with the brown solid (1-1-2) and 65 mL of phosphorus oxychloride, and the contents were heated and stirred at 120°C for one hour under a nitrogen atmosphere. The phosphorus oxychloride was distilled off under reduced pressure, and chloroform was added to the residue. They were then added to an aqueous sodium carbonate solution. The organic layer was concentrated, and the resulting concentrate was purified by column chromatography to obtain 1.7 g of a compound (1-1-3).

A flask was charged with 1.66 g of the compound (1-1-3) and 9.1 mL of pyrrolidine, and the contents were heated and stirred at 100°C for 30 minutes. The mixture was cooled to room temperature and concentrated, and the resulting concentrate was purified by column chromatography to obtain 1.47 g of a compound (1-1).

The following describes the result of ¹H-NMR measurement of the compound (1-1).
¹H-NMR (600 MHz, CDCl₃) δ 1.62 (s, 1H), 1.93 (brs, 2H), 1.98-2.14 (m, 2H), 2.87 (brs, 2H), 3.75 (brs, 2H), 6.93 (d, J = 5.58 Hz, 1H), 7.36-7.40 (m, 1H), 8.18-8.21 (m, 1H), 8.68 (d, J = 5.19 Hz, 1H).

### (Example 2) Synthesis of compound (1-2)

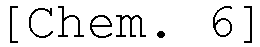

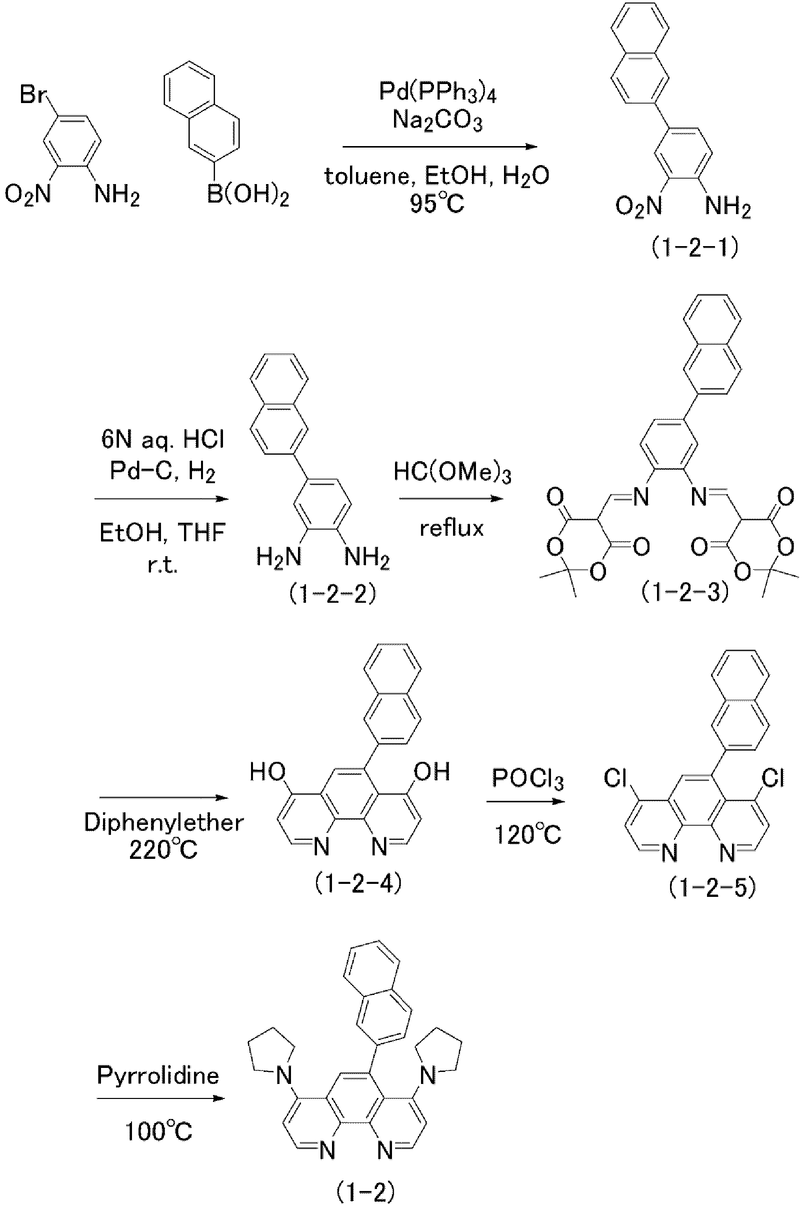

A flask was charged with 10 g of 4-bromo-2-nitroaniline, 8.7 g of 2-naphthaleneboronic acid, 1.06 g of Pd(PPh₃)₄, 14.7 g of sodium carbonate, 100 mL of toluene, 100 mL of distilled water, and 50 mL of ethanol, and the contents were stirred at 90°C for six hours. The reaction liquid was cooled to room temperature, water and heptane were added thereto, and the contents were stirred. The precipitated solid was collected by filtration, rinsed with water, methanol, and diethyl ether, and then dried in vacuum to obtain 12.0 g of a brown solid (1-2-1).

A flask was charged with 9.0 g of the brown solid (1-2-1), 0.45 g of 10% Pd-C, 90 mL of ethanol, and 60 mL of THF. Then, 11.3 mL of 6N hydrochloric acid was added thereto with stirring, and the contents were stirred for 36 hours under a hydrogen atmosphere. Water was added to the resulting suspension, and the pH was adjusted to 10 with a 4 N aqueous sodium hydroxide solution. Extraction was carried out with ethyl acetate. A combined organic layer was washed with water and saturated saline, and dried over potassium carbonate powder. After filtration and concentration, 9.4 g of a reddish brown solid (1-2-2) was obtained.

A flask was charged with 12.8 mmol of Meldrum's acid and 133 mL of methyl orthoformate, and the contents were heated and stirred at 130°C for two hours. The contents were cooled to 70°C, 9.4 g of the solid (1-2-2) was added thereto, and the contents were heated and stirred at 130°C for two hours. After the contents were cooled to room temperature, the precipitated solid was collected by filtration, rinsed with diethyl ether, and dried under vacuum to obtain 17.4 g of a compound (1-2-3). The compound (1-2-3) was gradually added to diphenyl ether heated to 240°C, and the contents were heated and stirred for 30 minutes. After the contents were cooled to room temperature, acetone was added thereto, and the precipitated solid was collected by filtration and rinsed with acetone to obtain 3.2 g of a brown solid (1-2-4).

A flask was charged with the solid (1-2-4) and 50 mL of phosphorus oxychloride, and the contents were heated and stirred at 100°C for one hour under a nitrogen atmosphere. The phosphorus oxychloride was distilled off under reduced pressure, and chloroform was added to the residue. They were then added to an aqueous sodium carbonate solution. The organic layer was concentrated, and the resulting concentrate was purified by column chromatography to obtain 1.7 g of a compound (1-2-5).

A flask was charged with 1.66 g of the compound (1-2-5) and 9.1 mL of pyrrolidine, and the contents were heated and stirred at 100°C for 30 minutes. The mixture was cooled to room temperature and concentrated, and the resulting concentrate was purified by column chromatography to obtain 1.28 g of a compound (1-2).

The following describes the result of ¹H-NMR measurement of the compound (1-2).
¹H-NMR (600 MHz, CDCl₃) δ 2.07-2.15 (m, 4H), 2.96 (brs, 4H), 3.77-3.84 (m, 4H), 6.73 (d, J = 5.58 Hz, 1H), 6.94 (d, J = 4.99 Hz, 1H), 7.45-7.52 (m, 2H), 7.58 (dd, J = 8.51, 1 76 Hz, 1H) 7.77 (d, J = 8.51 Hz, 1H), 7.83-7.90 (m, 2H), 7.96 (s, 1H), 7.99 (s, 1H), 8.74 (d, J = 5.58 Hz, 1H), 8.84 (d, J = 5.28 Hz, 1H).

### (Example 3) Synthesis of compound (1-3)

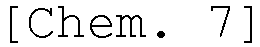

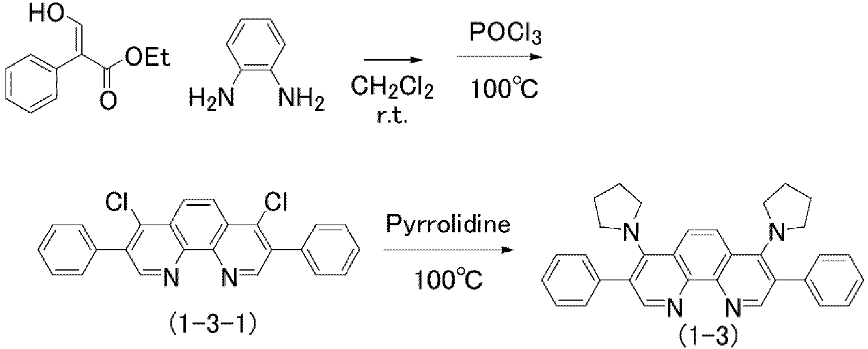

A flask was charged with 10 g of methyl 2-formyl-2-phenylacetate, 2.73 g of 1,2-phenylenediamine, and 76 mL of dichloromethane. The contents were stirred at room temperature overnight. The reaction solution was concentrated, 50 mL of phosphorus oxychloride was added to the resulting concentrate, and they were heated and stirred at 100°C for five hours. The phosphorus oxychloride was distilled off under reduced pressure, and chloroform was added to the residue. They were washed with an aqueous sodium carbonate solution for liquid-liquid separation. The organic layer was concentrated, and the resulting concentrate was purified by column chromatography to obtain **3.6** g of a compound (1-3-1).

A flask was charged with the compound (1-3-1) and 100 mL of pyrrolidine, and the contents were stirred at 100°C overnight. After the contents were cooled to room temperature, water was added thereto, and the precipitated solid was collected by filtration and dissolved in chloroform. The solution was transferred to a separatory funnel, washed with water and saturated saline, and then dried over sodium sulfate. The mixture was filtered, the solution was concentrated, and the resulting concentrate was purified by column chromatography to obtain 1.8 g of a compound (1-3).

The following describes the result of ¹H-NMR measurement of the compound (1-3).
¹H-NMR (600 MHz, CDCl₃) δ 1.78-2.04 (m, 4H), 3.10-3.44 (m, 4H), 7.34-7.56 (m, 3H), 7. 43 (brd, J = 1.47 Hz, 1H), 7.44 (brs, 1H), 8.01 (s, 1H), 8.84 (s, 1H)

### (Example 4) Synthesis of compound (1-4)

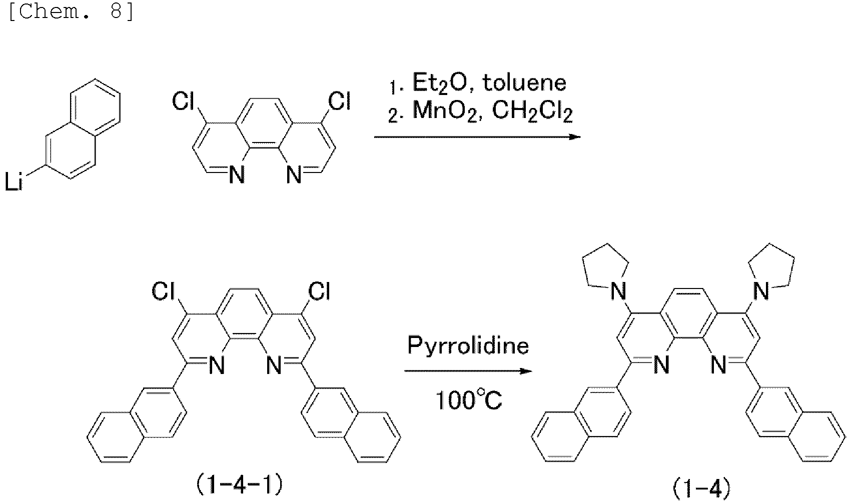

A flask was charged with 16.6 mmol of 2-bromonaphthalene and 100 mL of diethyl ether. Then, 100 mL of tert-butyllithium was added dropwise thereto at -78°C. After the dropwise addition was completed, the contents were stirred at the same temperature for one hour, and then 500 mL of toluene was added thereto. Then, 5.0 g of 4,7-dichlorophenanthroline was added thereto, and the contents were heated to 0°C and stirred overnight. Water was added thereto, extraction was performed with chloroform, and the organic layer was dried over sodium sulfate. The dried solution was filtered and concentrated. The resulting concentrate was dissolved in 300 mL of dichloromethane, 52.4 g of manganese dioxide was added thereto, and the contents were stirred at room temperature for two hours. The mixture was filtered through celite, and the filtrate was concentrated. The resulting concentrate was passed through a silica gel short column, and the resulting fraction was concentrated. Methanol was added to the resulting concentrate, and the precipitated solid was collected by filtration. The solid was rinsed with methanol to obtain 7.5 g of a compound (1-4-1).

A flask was charged with the compound (1-4-1) and 100 mL of pyrrolidine, and the contents were stirred at 100°C overnight. After the contents were cooled to room temperature, water was added thereto, and the precipitated solid was collected by filtration and rinsed with methanol and then with chloroform to obtain 5.0 g of a compound (1-4).

The following describes the result of ¹H-NMR measurement of the compound (1-4).
¹H-NMR (600 MHz, CDCl₃) δ 2.14 (dt, J = 6.16, 3.37 Hz, 4H), 3.82-3.88 (m, 4H), 7.43-7.46 (m, 1H) 7.51-7.57 (m, 2H), 7.92 (d, J = 7.92 Hz, 1H), 8.02-8.09 (m, 3H), 8.69 (dd, J = 8.51, 1.47 Hz, 1H), 8.96(s, 1H)

### (Example 5) Compound (1-5)

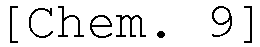

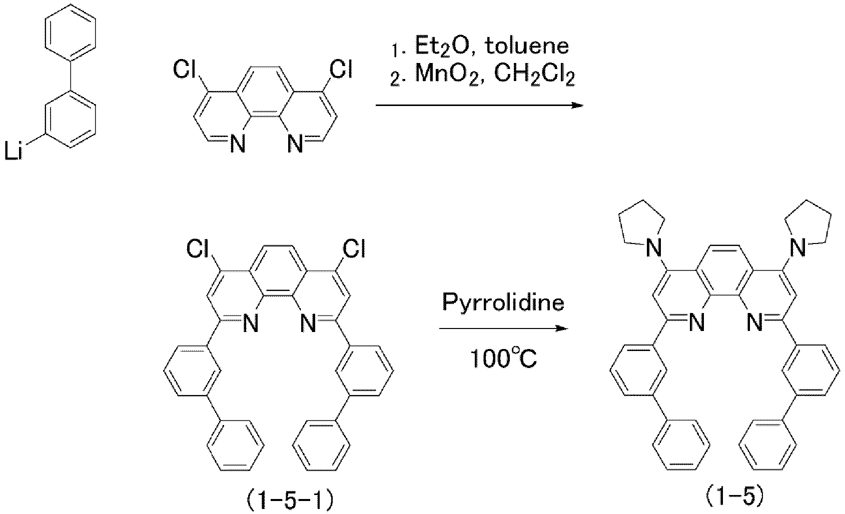

A flask was charged with 2.1 g of 3-bromobiphenyl and 10 mL of diethyl ether, and 3.3 mL of n-butyllithium was added dropwise thereto at -78°C. After the dropwise addition was completed, the contents were stirred at the same temperature for one hour, and then 50 mL of toluene was added thereto. Then, 0.55 g of 4,7-dichlorophenanthroline was added thereto, and the contents were heated to 0°C and stirred overnight. Water was added thereto, extraction was performed with chloroform, and the organic layer was dried over sodium sulfate. The dried solution was filtered and concentrated. The resulting concentrate was dissolved in 30 mL of dichloromethane, 5.8 g of manganese dioxide was added thereto, and the contents were stirred at room temperature for two hours. The mixture was filtered through celite, and the filtrate was concentrated. The resulting concentrate was passed through a silica gel short column, and the resulting fraction was concentrated. Methanol was added to the resulting concentrate, and the precipitated solid was collected by filtration. The solid was rinsed with methanol to obtain 1.15 g of a compound (1-5-1).

The compound was dissolved in 3.0 g of pyrrolidine and stirred at 100°C overnight. After the contents were cooled to room temperature, water was added thereto, and the precipitated solid was collected by filtration and rinsed with methanol to obtain 0.74 g of a compound (1-5).

The following describes the result of ¹H-NMR measurement of the compound (1-5).

¹H-NMR (600 MHz, CDCl₃) δ 8.65 (t, J = 1.6 Hz, 2H), 8.53-8.42 (m, 2H), 8.00 (s, 2H), 7.79 (dd, J = 1.0, 8.1 Hz, 4H), 7.68 (td, J = 1.2, 7.8 Hz, 2H), 7.60 (t, J = 7.6 Hz, 2H), 7.47 (t, J = 7.7 Hz, 4H), 7.42-7.35 (m, 2H), 7.32 (s, 2H), 3.87-3.75 (m, 8H), 2.18-2.04 (m, 8H)

### (Example 6) Synthesis of compound (1-6)

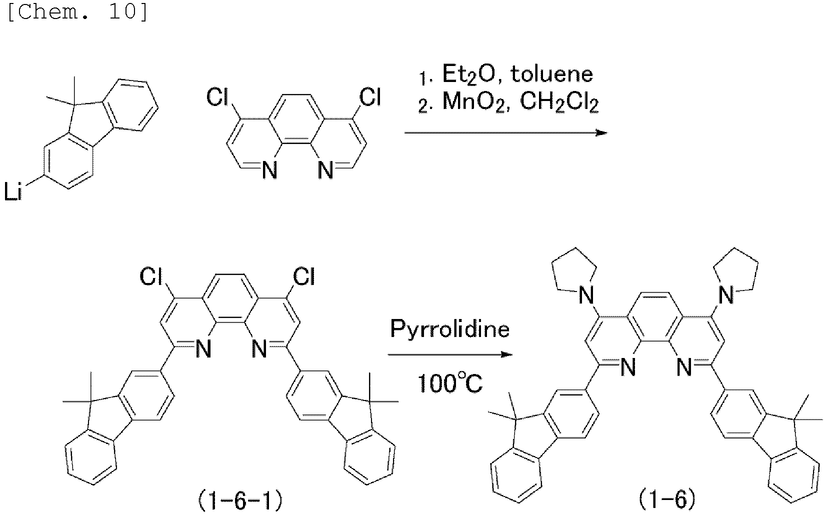

A compound (1-6) was synthesized as in Example 5, except that 2.4 g of 2-bromo-9,9-dimethylfluorene was used instead of 3-bromobiphenyl. The yield was 0.63 g.

The following describes the result of ¹H-NMR measurement of the compound (1-6).
¹H-NMR (600 MHz, CDCl₃) δ 8.58 (s, 2H), 8.32 (dd, J = 1.5, 7.9 Hz, 2H), 8.00 (s, 2H), 7.86 (d, J = 7.9 Hz, 2H), 7.81 (d, J = 6.7 Hz, 2H), 7.50 (d, J = 6.7 Hz, 2H), 7.43-7.31 (m, 2H), 7.33 (s, 2H), 6.98 (s, 2H), 3.81 (brt, J = 6.3 Hz, 8H), 2.18-2.06 (m, 8H), 1.43 (s, 12H)

### (Comparative Example 1) Synthesis of Comparative Compound 1 (4,7-di(pyrrolidin-1-yl)-1,10-phenanthroline)

4,7-Di(pyrrolidin-1-yl)-1,10-phenanthroline was obtained with reference to Synthesis Example 4 of Patent Literature 1 (WO 2021/045179).

### (Comparative Example 2) Synthesis of Comparative Compound 2 (2,9-butyl-4,7-di(pyrrolidin-1-yl)-1,10-phenanthroline)

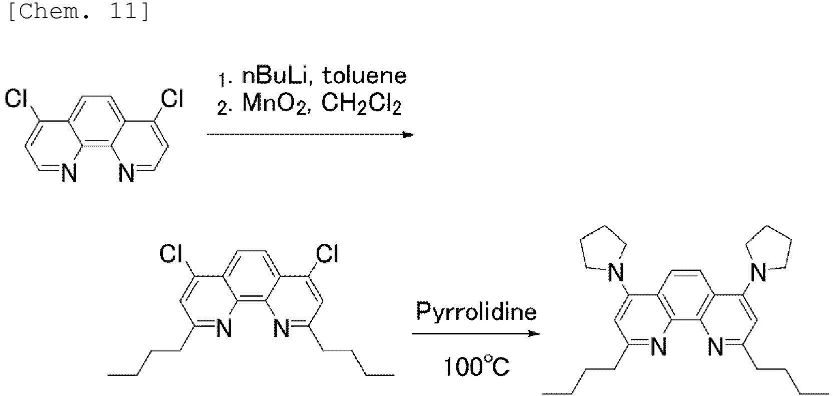

To a suspension of 3.0 g of 4,7-dichlorophenanthroline in 120 mL of toluene was added dropwise 30 mL of n-butyllithium at 0°C under a nitrogen atmosphere while stirring. The contents were stirred at the same temperature overnight, quenched by adding water, and then subjected to extraction with chloroform. The organic layer was washed with saturated saline and dried over sodium sulfate. The dried solution was filtered and concentrated. The resulting concentrate was dissolved in 120 m of dichloromethane, 12 g of manganese dioxide was added thereto, and the contents were stirred at room temperature for two hours. The mixture was filtered, the filtrate was concentrated, and the resulting concentrate was purified by column chromatography to obtain 3.0 g of 2,9-dibutyl-4,7-dichlorophenanthroline.

The obtained 2,9-dibutyl-4,7-dichlorophenanthroline was dissolved in 50 mL of pyrrolidine. The solution was stirred overnight at 100°C and then poured into water, and the precipitated solid was collected by filtration. The collected solid was purified by column chromatography to obtain 2.4 g of 2,9-butyl-4,7-di(pyrrolidin-1-yl)-1,10-phenanthroline as a comparative compound 2.

The following describes the result of ¹H-NMR measurement of the obtained 2,9-butyl-4,7-di(pyrrolidin-1-yl)-1,10-phenanthroline.
¹H-NMR (600 MHz, CDCl₃) δ 1.00 (t, J = 7.34 Hz, 3H), 1.52 (sxt, J = 7.39 Hz, 2H), 1.84-1.91 (m, 2H), 2.01-2.08 (m, 4H), 3.04-3.09 (m, 2H), 3.62-3.69 (m, 4H), 6.65 (s, 1H), 7.87 (s, 1H)

Table 1 shows the glass transition temperatures and melting points of the phenanthroline compounds of the present invention synthesized in Examples 1 to 6 and the comparative compounds 1 and 2 synthesized in Comparative Examples 1 and 2.

**[Table 1]**

| | Compound | Glass transition temperature (°C) | Melting point (°C) |
|---|---|---|---|
| Example 1 | 1-1 | 114 | 281 |
| Example 2 | 1-2 | 117 | 288 |
| Example 3 | 1-3 | 95 | 231 |
| Example 4 | 1-4 | 139 | 302 |
| Example 5 | 1-5 | 115 | 290 |
| Example 6 | 1-6 | 163 | 310 |
| Comparative Example 1 | Comparative compound 1 | 75 | 211 |
| Comparative Example 2 | Comparative compound 2 | 21 | 140 |

### (Example 7) Preparation of organic EL device 1

An organic EL device was prepared by the following method.

A transparent glass substrate having an ITO pattern with a thickness of 50 nm formed as an anode was prepared, rinsed by a common method, and then dried.

On the ITO of the substrate, a hole injection layer with a thickness of 30 nm, a hole transport layer with a thickness of 65 nm, an electron blocking layer with a thickness of 5 nm, a light-emitting layer with a thickness of 22 nm, a hole blocking layer with a thickness of 10 nm, and an electron transport layer with a thickness of 30 nm were formed in this order.

Furthermore, a film of the compound (1-1) of the present invention with a thickness of 1 nm was formed as an electron injection layer. A cathode made of silver and magnesium with a thickness of 100 nm was formed thereon. Here, the amount of silver was set to 10% by mass based on the entire amount of the cathode. Finally, the substrate on which the aforementioned layers to the cathode were formed was sealed to obtain the organic EL device 1.

### (Examples 8 to 10, Comparative Examples 3 and 4)

### Preparation of organic EL devices 2 to 6

Organic EL devices 2 to 6 were prepared as in Example 7, except that each electron injection layer included the compound (1-2), (1-3), (1-4), 8-hydroxyquinolinolatolithium (Liq), or the comparative compound 1.

The organic EL devices 1 to 6 were measured for driving voltage, luminance, current efficiency, and lifetime by the following methods. The evaluation results are shown in Table 2.

### <Measurement of emission characteristics of organic device>

A voltage was applied to the device using "6240 series source meter" available from ADC Corporation, and the luminance was measured using "BM-9" available from Topcon Corporation.

The current efficiency was calculated from the luminance and current value at the voltage applied to the device.

The lifetime corresponds to the time elapsed until the initial luminance of 1000 cd/cm² is reduced by 90%.

**[Table 2]**

| | Device | Electron injection layer | Driving voltage (V) | Luminance (cd/cm²) | Current efficiency (cd/A) | Lifetime (hour) |
|---|---|---|---|---|---|---|
| Example 7 | 1 | (1-1) | 4.5 | 576 | 5.87 | 172 |
| Example 8 | 2 | (1-2) | 4.4 | 612 | 6.15 | 148 |
| Example 9 | 3 | (1-3) | 4.5 | 593 | 6.02 | 180 |
| Example 10 | 4 | (1-4) | 4.5 | 592 | 5.98 | 177 |
| Comparative Example 3 | 5 | Liq | 4.4 | 605 | 6.08 | 152 |
| Comparative Example 4 | 6 | Comparative compound 1 | 4.5 | 596 | 6.08 | 168 |

The results in Table 1 show that the glass transition temperatures of the phenanthroline compounds of the present invention are at least 20°C or higher than those of Comparative Compound **1,** which is a phenanthroline compound having no substituent, and the comparative compound having an alkyl group as a substituent.

The results in Table 2 show that the organic EL devices using the phenanthroline compounds of the present invention have properties at the same level as those of Liq, which is a commonly used electron injection material, and the comparative compound **2.** Therefore, use of the phenanthroline compounds of the present invention allows organic EL devices to obtain improved durability while maintaining good properties.

### INDUSTRIAL APPLICABILITY

The organic thin film of the present invention can be used in organic electroluminescent devices, displays, lighting systems, organic thin-film solar cells, photoelectric conversion devices, thin-film transistors, and the like.

### REFERENCE SIGNS LIST

1: Organic EL device
2: Substrate
3: Anode
4: Hole injection layer
5: Hole transport layer
6: Light-emitting layer
7: Electron transport layer
8: Electron injection layer
9: Cathode
10: Oxide layer

## Claims

1. A phenanthroline compound represented by the following formula (1): 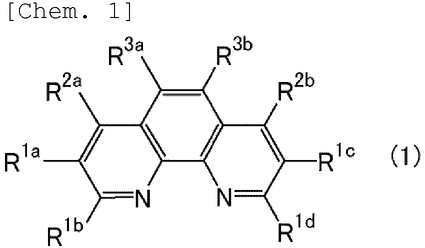 wherein R^{1a} to R^{1d} each independently represent a hydrogen atom, an aromatic hydrocarbon group optionally having a substituent, or an aromatic heterocyclic group optionally having a substituent; R^{2a} and R^{2b} each independently represent a dialkylamino group optionally having a substituent, and the dialkylamino group optionally having a substituent may form a ring structure; R^{3a} and R^{3b} each independently represent a hydrogen atom, an aromatic hydrocarbon group optionally having a substituent, an aromatic heterocyclic group optionally having a substituent, -R⁴=R⁵-R⁶ (where R⁴ and R⁵ each represent CH or N and R⁶ represents a hydrogen atom, an aromatic hydrocarbon group optionally having a substituent, or an aromatic heterocyclic group optionally having a substituent), or - R⁷-R⁸ (where R⁷ represents CH₂, NH, O, or S and R⁸ represents a hydrogen atom, an aromatic hydrocarbon group optionally having a substituent, or an aromatic heterocyclic group optionally having a substituent), or R^{3a} and R^{3b} are bonded to each other to form a fused ring structure; and at least one of R^{1a} to R^{1d}, R^{3a}, and R^{3b} is an aromatic hydrocarbon group optionally having a substituent or an aromatic heterocyclic group optionally having a substituent.

2. An organic thin film comprising the phenanthroline compound according to claim 1.

3. An organic semiconductor device material, comprising the phenanthroline compound according to claim 1.

4. The organic semiconductor device material according to claim 3,
wherein the organic semiconductor device material is for use in an organic semiconductor device selected from the group consisting of an organic electroluminescent device, an organic transistor, a photoelectric conversion device, and an organic solar cell.

5. An organic semiconductor device comprising the phenanthroline compound according to claim 1.

6. The organic semiconductor device according to claim 5,
wherein the organic semiconductor device is selected from the group consisting of an organic electroluminescent device, an organic transistor, a photoelectric conversion device, and an organic solar cell.

7. An image display or lighting system comprising the organic semiconductor device according to claim 5 or 6.
